(19) Europäisches Patentamt
European Patent Office
Office européen des brevets

(11) EP 2 764 883 B1

(12) **EUROPEAN PATENT SPECIFICATION**

(45) Date of publication and mention of the grant of the patent:
**22.02.2017 Bulletin 2017/08**

(21) Application number: **12838645.5**

(22) Date of filing: **05.10.2012**

(51) Int Cl.:
***A61M 5/28*** *(2006.01)* ***A61J 1/05*** *(2006.01)*
***C08G 63/199*** *(2006.01)*

(86) International application number:
**PCT/JP2012/075909**

(87) International publication number:
**WO 2013/051686 (11.04.2013 Gazette 2013/15)**

(54) **PACKAGING CONTAINER FOR MEDICAL USE**

VERPACKUNGSBEHÄLTER FÜR MEDIZINISCHE ZWECKE

CONDITIONNEMENT À USAGE MÉDICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.10.2011 JP 2011222600**
**13.01.2012 JP 2012005031**
**24.04.2012 JP 2012099155**

(43) Date of publication of application:
**13.08.2014 Bulletin 2014/33**

(60) Divisional application:
**15176619.3 / 2 946 802**

(73) Proprietor: **Mitsubishi Gas Chemical Company, Inc.**
**Tokyo 100-8324 (JP)**

(72) Inventors:
- **OGAWA, Shun**
**Hiratsuka-shi**
**Kanagawa 254-0016 (JP)**
- **HIROKANE, Takeshi**
**Hiratsuka-shi**
**Kanagawa 254-0016 (JP)**
- **KASHIBA, Takashi**
**Hiratsuka-shi**
**Kanagawa 254-0016 (JP)**
- **ARAKAWA, Shota**
**Hiratsuka-shi**
**Kanagawa 254-0016 (JP)**
- **USUDA, Kenichiro**
**Hiratsuka-shi**
**Kanagawa 254-0016 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 095 837**
**WO-A1-2008/075639**
**JP-A- H08 127 641**
**JP-A- 2001 240 661**
**JP-A- 2006 232 897**
**JP-A- 2007 238 856**
**JP-A- 2007 302 327**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 764 883 B1

## Description

### Technical Field

The present invention relates to a medical packaging container. More specifically, the present invention relates to a medical packaging container to be preliminarily filled with a drug solution for storage in a hermetically sealed state.

The present invention also relates to a manufacturing method of a polyester resin.

### Background Art

An ampoule, a vial, and a prefilled syringe, and the like are used as a medical packaging container to be filled with a drug solution for storage in a hermetically sealed state. Conventionally, these have been made from glass. A glass container, however, has disadvantages such as drop fragility and a large specific gravity which makes a medical packaging container heavy. A glass container also has disadvantages such as alkaline (Na+) elution into a liquid filling the container, generation of fine materials referred to as flakes, and possibility of contamination of the content with a metal for coloring in the case of using a light blocking colored glass container, during storage of the container filled with a drug solution. In view of the foregoing, there exists a need for replacing a medical packaging container made of glass with a medical packaging container made of plastic.

Examples of the characteristics required for a medical packaging container such as an ampoule, a vial, and a prefilled syringe include transparency and mechanical strength as a matter of course, high temperature resistance to endure a high temperature sterilization treatment, sterilization resistance to endure a high temperature sterilization treatment and a radiation sterilization treatment, water vapor barrier properties for preventing dissipation of water, oxygen barrier properties for preventing oxidation of a drug solution typified by protein, low ability to adsorb a drug solution for preventing adsorption of a drug solution typified by protein, and moldability.

For example, polycarbonate, polypropylene, a cycloolefin polymer or the like has been examined as a plastic for replacing glass for a prefilled syringe. The water vapor barrier properties, the oxygen barrier properties, and the ability to adsorb a drug solution are, however, insufficient for fulfilling the requirements, so that the replacement has not been commonly performed until now. Specifically, polycarbonate has the following problem: water of a drug solution volatilizes due to the insufficient water vapor barrier properties, and a prefilled syringe of polypropylene or a cycloolefin polymer has the following problems: a drug solution is oxidized due to insufficient oxygen barrier properties and a specific component of the drug solution is diluted due to the insufficiently low ability to adsorb a drug solution. The same problems occur for use in an ampoule and a vial.

It is known that a polyester resin has excellent oxygen barrier properties and low ability to adsorb a protein. Although polyethylene naphthalate (hereinafter sometimes referred to as PEN) also has water vapor barrier properties, it is crystalline, not functioning well as a syringe due to dimensional changes caused by partial crystallization during boiling disinfection. We have proposed a prefilled syringe made of a PEN resin copolymerized with a specific glycol in Patent Literature 1.

In Patent Literature 2, a prefilled syringe having a gasket of butyl rubber is disclosed, having an outer cylinder of polypropylene or cyclic polyolefin which is described therein.

In Patent Literature 3, a polyester polymer composed of dicarboxylic acid and diols including tricyclodecane dimethanols is disclosed, having use for a medical instrument material such as a syringe which is described therein.

In Patent Literature 4, a medical container made of polyolefin resin material is disclosed.

In Patent Literature 5, a medical container made of polyester resin material is disclosed.

In Patent Literature 6, a prefilled syringe having a multi-layer structure including the outermost layer and the innermost layer of a barrel made of a polyolefin resin, and the intermediate layer made of a resin having excellent barrier properties is disclosed.

A polyethylene terephthalate resin, i.e. one of a thermoplastic polyester resin (hereinafter sometimes referred to as PET), has excellent transparency, mechanical strength, melt stability, solvent resistance, aroma retention, and recyclability, being widely used as a film, a sheet, and a hollow container. The glass transition temperature of PET is, however, not necessarily sufficiently high, so that modification by copolymerization is widely performed. In addition, PET has the following problem: the transparency is impaired due to the crystallinity in forming a thick molded product.

In recent years, PEN has attracted attention among thermoplastic polyesters due to higher heat resistance than PET, excellent mechanical properties, barrier properties against oxygen and the like, and has been increasingly used in various applications including films and containers, in particular. However, PEN also has the following problem: the transparency is impaired due to the crystallinity in forming a thick molded product, so that modification by copolymerization has been examined.

For example, since tricyclodecane dimethanol (hereinafter sometimes referred to as TCDDM) and pentacyclopentadecane dimethanol (hereinafter sometimes referred to as PCPDM) are bulky and have rigid skeletons, a polyester

resin obtained by copolymerizing these has a high glass transition temperature, so that the transparency of a molded body may be improved due to the reduced crystallinity (with reference to Patent Literature 7 and 8).

In Patent Literature 9, a manufacturing method of a copolymerized polyester of terephthalic acid, ethylene glycol, and 1,4-cyclohexanedimethanol is disclosed, in which a polycondensation catalyst such as titanium, germanium, and antimony, and a toner such as cobalt are used in order to improve the transparency and the like.

Citation List

Patent Literature

Patent Literature 1: International Publication No. WO 2008/75639
Patent Literature 2: Japanese Patent Laid-Open No. 2004-298220
Patent Literature 3: Japanese Patent Laid-Open No. 2001-240661
Patent Literature 4: Japanese Patent Laid-Open No. 2003-138074
Patent Literature 5: Japanese Patent Laid-Open No. 8-127641
Patent Literature 6: Japanese Patent Laid-Open No. 2004-229750
Patent Literature 7: Japanese Patent Laid-Open No. 2007-238856
Patent Literature 8: Japanese Patent Laid-Open No. 58-174419
Patent Literature 9: Japanese Patent Laid-Open No. 2000-504770

Summary of Invention

Technical Problem

Although improvement is observed in the boiling disinfection resistance, the water vapor barrier properties, the oxygen barrier properties, and the low ability to adsorb a protein of the resin of the Patent Literature 1, further improvement is required. In particular, it cannot be said that the low ability to adsorb a protein is sufficient.

The sterilization resistance, the low ability to adsorb a drug solution, the water vapor barrier properties, and the oxygen barrier properties of a prefilled syringe of Patent Literature 2 are insufficient.

The water vapor barrier properties, the oxygen barrier properties, and the low ability to adsorb a protein required for a prefilled syringe to be filled with a drug solution for storage in a hermetically sealed state are not described in Patent Literature 3.

A container made of a polyolefin resin material as described in Patent Literature 4 has the following problems: a drug solution is oxidized due to insufficient oxygen barrier properties and that a specific component of a drug solution is diluted due to the ability to adsorb a drug solution, though having excellent water vapor barrier properties.

The medical container of Patent Literature 5 has inferior water vapor barrier properties compared to a container made of a polyolefin resin, so that volatilization of water from a drug solution may be caused, though having improved oxygen barrier properties.

The prefilled syringe of Patent Literature 6 has insufficiently improved ability to adsorb a drug solution, though having improved oxygen barrier properties.

In general, a copolymerized polyester is manufactured by polymerization using a polymerization catalyst such as an antimony compound and a titanium compound as well as in manufacturing of PET. The direct application of the manufacturing conditions of PET, however, causes problems such as insufficient polymerization activity and inferior color tone of a polymer in some cases.

For example, use of an antimony compound as a polycondensation catalyst alone or combined with a phosphorus thermal stabilizer allows a part of the antimony compound to be reduced to metal antimony, causing the following problem: the produced copolymerized polyester becomes dark in color. On the other hand, the decrease in the amount of the phosphorus thermal stabilizer for preventing the reduction of antimony compound causes the following problem: the produced copolymerized polyester becomes more yellowish in color, so that a polyester having high transparency cannot be obtained. It is therefore difficult to prevent the copolymerized polyester from becoming dark in color and yellowish in color at the same time.

It is known that use of a titanium compound as polycondensation catalyst causes the produced copolymerized polyester to become intensely yellowish in color, peculiar to a titanium catalyst. In the Patent Literature 9, a toner such as cobalt is added to improve the neutral color characteristics of copolymerized polyester. Inclusion of a toner such as cobalt in the copolymerized polyester causes the following problem: a produced copolymerized polyester becomes dark in color as in the case of using an antimony catalyst.

Copolymerized polyester of PEN copolymerized with TCDDM and/or PCPDM cannot sufficiently satisfy market needs for color tone, with the simple use of an antimony catalyst or a titanium catalyst as described above, though having excellent properties such as transparency, heat resistance, and low ability to adsorb contents.

An object of the present invention is to provide a medical packaging container having excellent mechanical strength, high temperature resistance, water vapor barrier properties, and oxygen barrier properties, with low ability to adsorb a drug solution.

Another object of the present invention is to provide a medical multi-layer container having an excellent water vapor barrier properties and oxygen barrier properties, with low ability to adsorb a drug solution.

Further another object of the present invention is to provide a manufacturing method of copolymerized polyester having an excellent color tone, preventing the copolymerized polyester from becoming dark in color and yellowish in color at the same time.

Solution to Problem

As a result of earnest research effort, the present inventors found that a medical packaging container made of polyester resin having structural units derived from specific glycol, in particular, has excellent performance, and accomplished the invention.

The present invention is as follows:

1. A medical packaging container comprising a polyester resin (A1) comprising diol units and dicarboxylic units, the diol units comprising at least one diol unit in an amount of 1 to 30 mol% selected from diol units having a bridged alicyclic skeleton derived from a compound represented by formula (1), formula (2), or formula (3), the dicarboxylic acid units comprising dicarboxylic acid units having a naphthalene skeleton in an amount of 70 mol% or more.

[Formula 1]

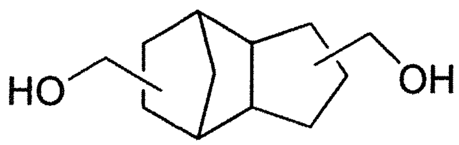

(1)

[Formula 2]

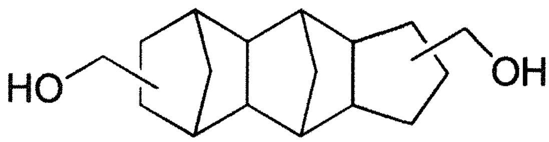

(2)

[Formula 3]

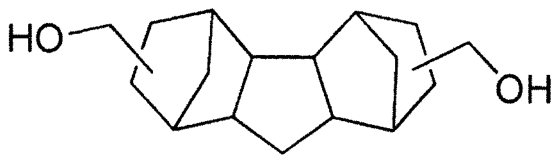

(3)

2. The medical packaging container according to 1., wherein the diol units comprise at least one diol unit in an amount of 1 to 30 mol% selected from diol units having a bridged alicyclic skeleton derived from a compound represented by the formula (1) or the formula (2).
3. The medical packaging container according to 1., wherein the diol units comprise diol units having a bridged alicyclic skeleton derived from a compound represented by the formula (1) in an amount of 1 to 30 mol%.
4. The medical packaging container according to any one of 1. to 3., wherein the dicarboxylic acid units having a

naphthalene skeleton are derived from at least one dicarboxylic acid unit selected from the group consisting of 1,3-naphthalenedicarboxylic acid, 1,4-naphthalenedicarboxylic acid, 1,5-naphthalenedicarboxylic acid, 2,6-naphthalenedicarboxylic acid, and 2,7-naphthalenedicarboxylic acid.

5. The medical packaging container according to any one of 1. to 4., wherein the dicarboxylic acid units comprise the dicarboxylic acid units having a naphthalene skeleton in an amount of 90 mol% or more.

6. The medical packaging container according to any one of 1. to 5., wherein the diol units comprise diol units derived from ethylene glycol.

7. The medical packaging container according to any one of 1. to 6., wherein the polyester resin (A1) has all of the following properties (i) to (iii):

(i) a glass transition temperature of 110°C or higher as measured with a differential scanning calorimeter;
(ii) a moisture permeability coefficient of 1 g·mm/m$^2$/day or less; and
(iii) an oxygen permeability coefficient of 10 cc·mm/m$^2$/day/atm or less.

8. The medical packaging container according to 7., wherein the polyester resin (A1) has the following property (iv):

(iv) an increase amount in nitrogen concentration of 5 ppm or less when a polyester resin film having a thickness of 100 μm is immersed in a 1 wt.% aqueous solution of albumin.

9. The medical packaging container according to any one of 1. to 8., wherein the polyester resin (A1) has the following property (v):

(v) a melt flow rate in accordance with JIS K 7210 of 1 to 40 g/10 minutes under conditions of 260°C and 2.16 kgf.

10. The medical packaging container according to any one of 1. to 9., wherein the polyester resin (A1) has a manganese atom content of 40 to 200 ppm and an antimony atom content of 50 to 200 ppm, with a ratio (M/P) of the total content (M) (ppm) of manganese atoms and antimony atoms to the phosphorus atom content (P) (ppm) of 1.5 to 4.0.

11. The medical packaging container according to 10., wherein the polyester resin (A1) is obtained by a manufacturing method comprising the steps of:

producing an oligomer by transesterification of a dicarboxylic acid diester component comprising naphthalenedicarboxylic acid diester and a diol component comprising at least one selected from the compounds represented by the formula (1), the formula (2), and the formula (3) in the presence of a manganese compound; and polycondensing the oligomer in the presence of an antimony compound; and

wherein, in the manufacturing method, a phosphorus compound is used as a thermal stabilizer.

12. The medical packaging container according to 11., wherein the diol component further comprises ethylene glycol.

13. The medical packaging container according to any one of 1. to 12., comprising a multi-layer structure having at least three layers, and
the innermost layer and the outermost layer in the multi-layer structure comprising the polyester resin (A1).

14. The medical packaging container according to 13., wherein at least one layer of the intermediate layers in the multi-layer structure comprises a polyolefin resin (B).

15. The medical packaging container according to 14., wherein the polyolefin resin (B) is at least one selected from cycloolefin polymer, cycloolefin copolymer, and polypropylene.

16. The medical packaging container according to any one of 1. to 15., wherein the medical packaging container is an ampoule, a vial, or a prefilled syringe.

Furthermore, as a result of earnest research effort, the present inventors found that copolymerized polyester having an excellent color tone, which can be produced with use of a specific amount of an antimony compound and a manganese compound as catalysts and a specific amount of phosphorus compound as a thermal stabilizer, and accomplished the invention.

The present invention is as follows:

17. A manufacturing method of a polyester resin, comprising the steps of:

producing an oligomer by transesterification of a dicarboxylic acid diester component comprising naphthalenedicarboxylic acid diester and at least one selected from the compounds represented by the formula (1), the

formula (2), and the formula (3), and a diol component comprising ethylene glycol in the presence of a manganese compound; and
polycondensing the oligomer in the presence of an antimony compound;

wherein a phosphorus compound is used as a thermal stabilizer, and
wherein the polyester resin has a manganese atom content of 40 to 200 ppm and an antimony atom content of 50 to 200 ppm, with a ratio (M/P) of the total content (M) (ppm) of manganese atoms and antimony atoms to the phosphorus atom

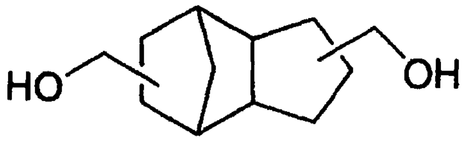

(1)

content (P) (ppm) of 1.5 to 4.0.

[Formula 6]

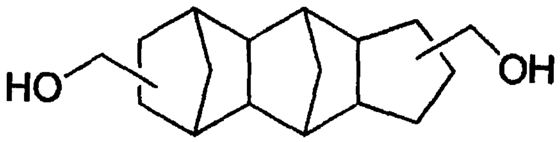

(2)

[Formula 7]

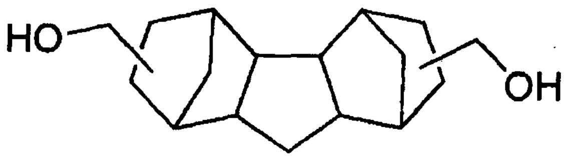

(3)

[Formula 8]

18. The manufacturing method of the polyester resin according to 17., wherein the manganese atoms are derived from manganese acetate, the antimony atoms are derived from antimony trioxide, and phosphorus atoms are derived from phosphoric acid.
19. The manufacturing method of the polyester resin according to 17. or 18., wherein the ratio of ethylene glycol to the total of the compounds represented by the formula (1), the formula (2), and the formula (3) in the diol components is 70 to 99 mol%/30 to 1 mol%.
20. The manufacturing method of the polyester resin according to any one of 17. to 19., wherein the naphthalenedicarboxylic acid diester is dimethyl 2,6-naphthalenedicarboxylate.

Advantageous Effects of Invention

The present invention can provide a medical packaging container having excellent mechanical strength, high temperature resistance, water vapor barrier properties, and oxygen barrier properties, with low ability to adsorb a drug solution.

Further, the present invention can provide a medical multi-layer container having an excellent water vapor barrier properties and oxygen barrier properties, with low ability to adsorb a drug solution.

Furthermore, the present invention can provide a manufacturing method of polyester resin having an excellent color tone, preventing the polyester from becoming dark in color and yellowish in color at the same time.

Description of Embodiments

The embodiments of the present invention are described in the following (hereinafter referred to as embodiments). The embodiments are given for illustrative purpose only, and the invention is not limited thereto.

[Medical packaging container]

<Polyester resin (A1)>

In an embodiment, a medical packaging container includes a polyester resin (A1) of which diol units include at least one diol unit in an amount of 1 to 30 mol% selected from diol units having a bridged alicyclic skeleton derived from a compound represented by formula (1), formula (2), or formula (3), and of which dicarboxylic acid units include dicarboxylic acid units having a naphthalene skeleton in an amount of 70 mol% or more.

[Formula 9]

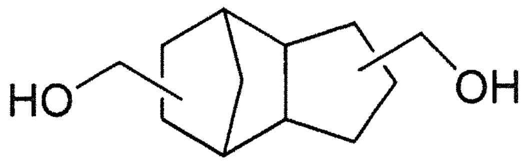

(1)

[Formula 10]

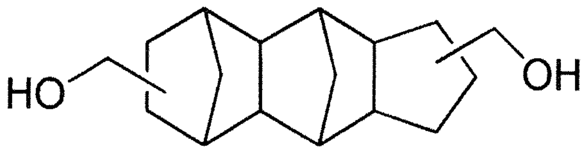

(2)

[Formula 11]

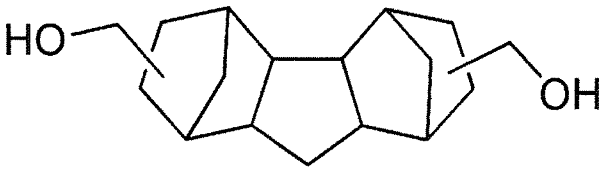

(3)

A polyester resin including at least one diol unit selected from diol units having a bridged alicyclic skeleton derived from a compound represented by formula (1), formula (2), or formula (3) in an amount of 1 mol% or more has an increased glass transition temperature, so that high temperature resistance of the polyester resin is improved. In addition, since the crystallinity is reduced, partial crystallization during boiling disinfection and dimensional changes, whitening, and embrittlement associated therewith can be suppressed. The proportion of the diol units having the bridged alicyclic skeleton is preferably 3 mol% or more, more preferably 5 mol% or more. Meanwhile, with a proportion of diol units having the bridged alicyclic skeleton of 30 mol% or less in the total diol units of a polyester resin, the polyester resin has improved water vapor barrier properties and oxygen barrier properties. In considering the high temperature resistance, the water vapor barrier properties, and the oxygen barrier properties of a polyester resin, the proportion of diol units having the bridged alicyclic skeleton is preferably 1 to 25 mol%, more preferably 3 to 20 mol%, furthermore preferably 5 to 15 mol%.

Specific examples of the compound represented by the formula (1) include, tricyclo [5.2.1.$0^{2,6}$] decane-3, 8-dimethanol, tricyclo[5.2.1.$0^{2,6}$]decane-3,9-dimethanol, tricyclo[5.2.1.$0^{2,6}$]decane-4,8-dimethanol, tricyclo[5.2.1.$0^{2,6}$] decane-4, 9-dimethanol, tricyclo[5.2.1.$0^{2,6}$]decane-4,8-dimethanol, and tricyclo[5.2.1.$0^{2,6}$]decane-4,9-dimethanol.

Specific examples of the compound represented by the formula (2) include, pentacyclo[6.5.1.$1^{3,6}$.$0^{2,7}$.$0^{9,13}$]pentadecane-4,10-dimethanol, pentacyclo[6.5.1.$1^{3,6}$.$0^{2,7}$.$0^{9,13}$]pentadecane-4,11-dimethanol, pentacyclo[6.5.1.$1^{3,6}$.$0^{2,7}$.$0^{9,13}$]pentadecane-4,12-dimethanol, pentacyclo[6.5.1.$1^{3,6}$.$0^{2,7}$.$0^{9,13}$]pentadecane-5,10-dimethanol, pentacyclo[6.5.1.1$^{3,6}$.0$^{2,7}$.0$^{9,13}$]pentadecane-5,11-dimethanol, and pentacyclo[6.5.1.1$^{3,6}$.0$^{2,7}$.0$^{9,13}$]pentadecane-5,12-dimethanol.

Specific examples of the compound represented by the formula (3) include, pentacyclo[9.2,1.1$^{4,7}$.0$^{2,10}$.0$^{3,8}$]pentadecane-5,12-dimethanol, pentacyclo[9.2.1.1$^{4,7}$.0$^{2,10}$.0$^{3,8}$]pentadecane-5,13-dimethanol, pentacyclo[9.2.1.1$^{4,7}$.0$^{2,10}$.0$^{3,8}$]pentadecane-6,12-dimethanol, and pentacyclo[9.2.1.1$^{4,7}$.0$^{2,10}$.0$^{3,8}$]pentadecane-6,13-dimethanol.

These may be used singly or in combinations of two or more at the same time.

The diol units having the bridged alicyclic skeleton is preferably at least one diol unit selected from diol units having a bridged alicyclic skeleton derived from a compound represented by the formula (1) or the formula (2), in considering the low ability to adsorb a drug solution, the high temperature resistance, and the water vapor barrier properties, and more preferably the diol unit having a bridged alicyclic skeleton derived from a compound represented by the formula (1), in considering economic efficiency and availability.

Examples of the diol units of the polyester resin (A1) other than diol units having a bridged alicyclic skeleton include, but not specifically limited to, aliphatic diols such as ethylene glycol, trimethylene glycol, 1,4-butanediol, 1,5-pentanediol, 1,6-hexanediol, diethylene glycol, propylene glycol, and neopentyl glycol; alicyclic diols such as 1,3-cyclohexanedimethanol, 1,4-cyclohexanedimethanol, 1,2-decahydronaphthalene dimethanol, 1,3-decahydronaphthalene dimethanol, 1,4-decahydronaphthalene dimethanol, 1,5-decahydronaphthalene dimethanol, 1,6-decahydronaphthalene dimethanol, 2,7-decahydronaphthalene dimethanol, and tetralin dimethanol; polyether compounds such as polyethylene glycol, polypropylene glycol, and polybutylene glycol; bisphenols such as 4,4'-(1-methylethylidene)bisphenol, methylene bisphenol (bisphenol F), 4,4'-cyclohexylidene bisphenol (bisphenol Z), and 4,4'-sulfonyl bisphenol (bisphenol S); alkylene oxide adducts of the bisphenols; an aromatic dihydroxy compound such as hydroquinone, resorcin, 4,4'-dihydroxybiphenyl, 4,4'-dihydroxydiphenyl ether, and 4,4'-dihydroxydiphenyl benzophenone; and diol units derived from alkylene oxide adducts of the aromatic dihydroxy compound. In considering the mechanical strength and the high temperature resistance of a polyester resin and the availability of a diol, a diol unit derived from ethylene glycol, trimethylene glycol, 1,4-butanediol, or 1,4-cyclohexanedimethanol is preferable, and a diol unit derived from ethylene glycol is more preferable. Diol units other than the diol units having a bridged alicyclic skeleton may be of one alone or consist of two or more selected from the above.

The proportion of dicarboxylic acid units having a naphthalene skeleton in the total dicarboxylic acid units of the polyester resin (A1) for use in the present embodiment is 70 mol% or more. The inclusion of dicarboxylic acid units having a naphthalene skeleton in an amount of 70 mol% or more allows for the increase in the glass transition temperature, i.e. the improvement in high temperature resistance, the improvement in water vapor barrier properties, and the improvement in oxygen barrier properties of the polyester resin (A1), at the same time. In considering the high temperature resistance, the water vapor barrier properties, and the oxygen barrier properties of the polyester resin, the proportion of dicarboxylic acid units having a naphthalene skeleton is preferably 80 mol% or more, more preferably 90 mol% or more, furthermore preferably 95 mol% or more, and particularly preferably 100 mol%.

In considering the reactivity of polycondensation, the dicarboxylic acid unit having a naphthalene skeleton in the polyester resin (A1) for use in the present embodiment is preferably, but not specifically limited to, a unit derived from 1,3-naphthalenedicarboxylic acid, 1,4-naphthalenedicarboxylic acid, 1,5-naphthalenedicarboxylic acid, 2,6-naphthalenedicarboxylic acid, 2,7-naphthalenedicarboxylic acid, or the like. The dicarboxylic acid unit having a naphthalene skeleton may be of one alone or consist of two or more. In considering the high temperature resistance, the water vapor barrier properties, and the economic efficiency, a unit derived from 2,6-naphthalenedicarboxylic acid is most preferred among those described above.

The polyester resin (A1) for use in the present embodiment may have the dicarboxylic acid units other than the dicarboxylic acid units having a naphthalene skeleton. Examples of the dicarboxylic acid units other than the dicarboxylic acid units having a naphthalene skeleton include, but not specifically limited to, a unit derived from aliphatic dicarboxylic acid such as succinic acid, glutaric acid, adipic acid, pimelic acid, suberic acid, azelaic acid, sebacic acid, decane dicarboxylic acid, dodecane dicarboxylic acid, cyclohexane dicarboxylic acid, decalindicarboxylic acid, norbornane dicarboxylic acid, tricyclodecane dicarboxylic acid, pentacyclo dodecane dicarboxylic acid, 3,9-bis(1,1-dimethyl-2-carboxyethyl)-2,4,8,10-tetraoxaspiro[5.5]undecane, 5-carboxy-5-ethyl-2-(1,1-dimethyl-2-carboxyethyl)-1,3-dioxane, and dimer acid; and a unit derived from an aromatic dicarboxylic acid such as terephthalic acid, isophthalic acid, phthalic acid, 2-methyl terephthalic acid, biphenyl dicarboxylic acid, and tetralindicarboxylic acid. In considering the mechanical strength, the high temperature resistance of the polyester resin, a unit derived from aromatic dicarboxylic acid is preferred, and considering the availability of dicarboxylic acid, a unit derived from terephthalic acid or isophthalic acid is more preferred. The dicarboxylic acid unit other than the dicarboxylic acid units having a naphthalene skeleton of a polyester resin may be of one alone or consist of two or more.

For the adjustment of melt viscoelasticity and molecular weight and the like, a polyester resin (A1) for use in the present embodiment may include units derived from monoalcohol such as butyl alcohol, hexyl alcohol, and octyl alcohol; units derived from a multivalent alcohol of trivalent or more such as trimethylolpropane, glycerin, 1,3,5-pentane

triol, and pentaerythritol; units derived from a monocarboxylic acid such as benzoic acid, propionic acid, and butyric acid; units derived from a multivalent carboxylic acid of trivalent or more such as trimellitic acid and pyromellitic acid; and units derived from an oxyacid such as glycolic acid, lactic acid, hydroxybutyric acid, 2-hydroxy isolactic acid, and hydroxybenzoic acid, within a range not impairing the object of the present invention.

In particular, in considering the high temperature resistance, the water vapor barrier properties, the oxygen barrier properties, and the mechanical performance of a polyester resin (A1) for use in the present embodiment, the diol unit having a bridged alicyclic skeleton is preferably a unit derived from at least one diol selected from the group consisting of tricyclo[5.2.1.0$^{2,6}$]decane-3,8-dimethanol, tricyclo[5.2.1.0$^{2,6}$]decane-3,9-dimethanol, tricyclo[5.2.1.0$^{2,6}$]decane-4,8-dimethanol, and tricyclo[5.2.1.0$^{2,6}$]decane-4,9-dimethanol, while the diol unit other than the diol unit having a bridged alicyclic skeleton is preferably a unit derived from ethylene glycol, and the entire dicarboxylic acid units are preferably units derived from 2,6-naphthalenedicarboxylic acid.

In considering the high temperature resistance, the high temperature sterilization resistance, the water vapor barrier properties, and the oxygen barrier properties required for a medical packaging container, the polyester resin (A1) for use in the present embodiment has preferably all of the following properties (i) to (iii):

(i) a glass transition temperature of 110°C or higher as measured with a differential scanning calorimeter;
(ii) a moisture permeability coefficient of 1 g·mm/m$^2$/day or less; and
(iii) an oxygen permeability coefficient of 10 cc·mm/m$^2$/day/atm or less.

The glass transition temperature of the polyester resin (A1) for use in the present embodiment is measured with a differential scanning calorimeter (DSC). Namely, approximately 10 mg of polyester resin is placed in an unsealed container made of aluminum so as to be heated to 280°C in a nitrogen gas (30 ml/min) stream at a temperature increase rate of 20°C/min. The melted resin is quenched to form a measurement sample. The sample is measured under the same conditions and the temperature corresponding to the changes by 1/2 of the difference between the baselines before and after the transition in the DSC curve is defined as the glass transition temperature.

The glass transition temperature of the polyester resin (A1) for use in the present embodiment is preferably, but not specifically limited to, 110°C or higher, more preferably 115°C or higher, furthermore preferably 120°C or higher, as measured with a DSC. A polyester resin having a glass transition temperature in the range allows a medical packaging container in the present embodiment to have a further improved high temperature resistance to endure a boiling disinfection. The glass transition temperature of the polyester resin (A1) in the present embodiment can be adjusted into the range by properly selecting diols having the bridged alicyclic skeleton and carboxylic acids having a naphthalene skeleton. The upper limit of the glass transition temperature is preferably, but not specifically limited to, 160°C or lower, in considering the type and the composition of the constituent units of a polyester resin.

The water vapor barrier properties of the polyester resin (A1) for use in the present invention can be represented by moisture permeability coefficient. The moisture permeability coefficient is calculated from the following expression based on the water vapor permeation rate of a film having a thickness of 200 μm formed by melt extrusion as a measurement sample, under measurement conditions at 40°C and 90% RH:

$$\text{Moisture permeability coefficient } (\text{g}\cdot\text{mm/m}^2/\text{day}) = \text{Water vapor permeation rate } (\text{g/m}^2/\text{day}) \times \text{Thickness (mm)}$$

The moisture permeability coefficient of the polyester resin (A1) for use in the present embodiment is preferably, but not specifically limited to, 1 g·mm/m$^2$/day or less, more preferably 0.8 g·mm/m$^2$/day or less, furthermore preferably 0.6 g·mm/m$^2$/day or less. A moisture permeability coefficient of the polyester resin (A1) in the range allows the transpiration of moisture in a drug solution to be further suppressed, so that the long-term storage stability of the drug solution in a medical packaging container can be further improved. The moisture permeability coefficient of a polyester resin can be adjusted within the range by properly selecting diols having a bridged alicyclic skeleton and dicarboxylic acid having a naphthalene skeleton as described above.

The oxygen barrier properties of the polyester resin (A1) for use in the present invention can be represented by oxygen permeability coefficient. The oxygen permeability coefficient is calculated from the following expression based on the oxygen permeation rate of a film having a thickness of 200 μm formed by melt extrusion as a measurement sample, under measurement conditions at 23°C and 65% RH:

$$\text{Oxygen permeability coefficient } (cc{\cdot}mm/m^2/day/atm) = \text{Oxygen permeation rate } (cc/m^2/day/atm) \times \text{Thickness (mm)}$$

The oxygen permeability coefficient of the polyester resin (A1) for use in the present embodiment is preferably, but not specifically limited to, 10 cc·mm/$m^2$/day/atm or less, more preferably 5 cc·mm/$m^2$/day/atm or less, furthermore preferably 2 cc·mm/$m^2$/day/atm or less, particularly preferably 1.5 cc·mm/$m^2$/day/atm or less. A oxygen permeability coefficient of the polyester resin (A) in the range further prevents oxidation deterioration of a drug solution, so that the long-term storage stability of the drug solution in a thus produced medical packaging container can be further improved. The oxygen permeability coefficient of a polyester resin can be adjusted within the range by properly selecting diols having a bridged alicyclic skeleton and dicarboxylic acid having a naphthalene skeleton as described above.
In considering prevention of the change in concentration of a drug solution, preferably the polyester resin (A1) for use in the present embodiment further has the following properties (iv):

(iv) an increase in nitrogen concentration of 5 ppm or less when a film having a thickness of 100 μm is immersed in a 1 wt.% aqueous solution of albumin.

The increase in nitrogen concentration indicates the ability to adsorb a drug solution (protein) of the polyester resin (A1) for use in the present embodiment, so that the amount of adsorbed protein can be obtained from the amount of nitrogen in the protein. The increase in nitrogen concentration is calculated from the following expression based on the nitrogen concentration of a film having a thickness of 100 μm produced by melt extrusion, which is immersed in an aqueous solution of protein for 8 days at 23°C and 50% RH and washed with pure water 5 times after immersion for the determination by nitrogen element analysis, and the nitrogen concentration of a film before immersion, which is preliminarily measured before filling with the aqueous solution of protein:

$$\text{Increase in nitrogen concentration (ppm)} = (\text{Nitrogen concentration of film after immersion (ppm)}) - (\text{Nitrogen concentration of film before immersion (ppm)})$$

An 1 wt.% aqueous solution of albumin is used as the aqueous solution of protein.
The increase in nitrogen concentration of the polyester resin (A1) for use in the embodiment of the present invention is preferably, but not specifically limited to, 5 ppm or less, preferably 4 ppm or less, furthermore preferably 3 ppm or less. An increase in the nitrogen concentration of a polyester resin in the range further prevents adsorption to a drug solution, so that the long-term storage stability of the drug solution in a thus produced medical packaging container can be further improved. The increase in nitrogen concentration of a polyester resin can be adjusted within the range by properly selecting diols having a bridged alicyclic skeleton and dicarboxylic acid having a naphthalene skeleton as described above.
The polyester resin (A1) in the present embodiment has preferably the following properties (v):

(v) a melt flow rate (MFR) in accordance with JIS K 7210 of 1 to 40 g/10 minutes under conditions of 260°C and 2.16 kgf.

The melt viscosity can be represented by the melt flow rate (MFR) described in JIS K 7210, and the measurement value under conditions of 260°C and 2.16 kgf is preferably in the range of 1 to 40 g/10 minutes, more preferably 3 to 30 g/10 minutes, furthermore preferably 5 to 20 g/10 minutes. A melt viscosity in this range allows the polyester resin (A1) to have more excellent balance of moldability and mechanical performance.
The polyester resin (A1) in the present embodiment, which is manufactured by, for example, the following "manufacturing method of polyester", preferably has a manganese atom content of 40 to 200 ppm and an antimony atom content of 50 to 200 ppm, with a ratio (M/P) of the total content (M) (ppm) of manganese atoms and antimony atoms to the phosphorus atom content (P) (ppm) of 1.5 to 4.0.
Manganese atoms in the polyester resin (A1) are derived from an additive manganese compound as a catalyst for the transesterification reaction. In order to preserve the sufficient catalytic activity and prevent the polyester resin (A1) from becoming dark in color and yellowish in color, the manganese atom content is preferably 40 to 200 ppm, more preferably 45 to 150 ppm, furthermore preferably 50 to 100 ppm.
Antimony atoms in the polyester resin (A1) are derived from an additive antimony compound as a polycondensation catalyst. In order to preserve the sufficient polycondensation catalytic activity and prevent the polyester resin (A1) from becoming dark in color and yellowish in color, the antimony atom content is preferably 50 to 200 ppm, more preferably 60 to 150 ppm, furthermore preferably 70 to 100 ppm.

In considering the color tone, the polyester resin (A1) in the present embodiment is preferably obtained by a manufacturing method including the steps of producing an oligomer by the transesterification reaction of a dicarboxylic acid diester component containing naphthalenedicarboxylic acid diester and a diol component containing a compound represented by the formula (1), the formula (2), or the formula (3) in the presence of a manganese compound, and polycondensing the oligomer in the presence of an antimony compound, with use of a phosphorus compound as a thermal stabilizer.

Various kinds of additives and molding auxiliary agents such as an antioxidant, a light stabilizers, an ultraviolet absorber, a plasticizer, a bulking agent, a matting agent, a drying control agent, an antistatic agent, an antisettling agent, a surfactant, a flow modifier, a drying oil, waxes, a filler, a colorant, a reinforcing agent, a surface lubricant, a leveling agent, a curing accelerator, and a thickener may be added to the polyester resin (A1) for use in the present embodiment within a range not impairing the object of the present invention.

<Layer structure of packaging container>

The layer structure of a medical packaging container in the present embodiment is not particularly limited. The structure may be a single-layer structure or a multi-layer structure having at least three layers including the innermost layer and the outermost layer which contain the polyester resin (A1). The multi-layer structure includes the innermost layer and the outermost layer made of the polyester resin (A1), so that the effects such as low ability to adsorb a drug solution and high oxygen barrier properties can be provided.

[Multi-layer packaging container]

A packaging container according to the present disclosure includes a packaging container of a multi-layer structure, comprising a multi-layer structure having at least three layers including the innermost layer and the outermost layer which contain an amorphous polyester resin (A) and at least one layer of intermediate layers of which contains a polyolefin resin (B).

The multi-layer packaging container according to the present disclosure comprises a multi-layer structure including the innermost layer and the outermost layer each formed of a layer (layer A) containing an amorphous polyester resin (A) and at least one layer of intermediate layers (layer B) contains a polyolefin resin (B).

The innermost layer and the outermost layer contain the amorphous polyester resin (A), so that the multi-layer container according to the present disclosure has low ability to adsorb a drug solution and high oxygen barrier properties. At least one layer of the intermediate layers contains the polyolefin resin (B), so that the multi-layer container has high water vapor barrier.

The number and the type of the layer A and the layer B of the multi-layer packaging container according to the present disclosure are not particularly limited. Examples may include a three-layer A/B/A structure consisting of two layers A and one layer B and a five-layer A/B/A/B/A structure consisting of three layers A and two layers B. The multi-layer packaging container according to the present disclosure may include an optional layer such as an adhesion layer (AD) on an as needed basis. For example, a five-layer A/AD/B/AD/A structure may be employed.

[Layer A]

The layer A of a multi-layer packaging container according to the present disclosure contains an amorphous polyester resin (A). The amorphous polyester resin (A) contained in the layer A may be of one or in combinations of two or more.

The amorphous polyester resin (A) contained in the layer A constituting the innermost layer and the amorphous polyester resin (A) contained in the layer A constituting the outermost layer may be the same or different.

A resin other than the amorphous polyester resin (A) may be added to the Layer A corresponding to the effect to be imparted within a range not impairing the primary object.

Although the content of the amorphous polyester resin (A) in the layer A is not particularly limited, the layer A preferably includes the amorphous polyester resin (A) as a main component. The "main component" is not particularly limited so long as the effect of polyester resin (A) sufficiently exhibits, meaning that the content of polyester resin (A) in the layer A is, for example, 50 mass% or more.

The content of amorphous polyester resin (A) in the layer A is more preferably 60 mass%, furthermore preferably more than 90 mass%. The resin contained in the layer A may be the amorphous polyester resin (A) alone.

In order to ensure various physical properties required for a medical multi-layer container, the thickness of the

layer A is preferably 50 to 10,000 μm, more preferably 100 to 7,000 μm, furthermore preferably 300 to 5,000 μm.

<Amorphous polyester resin (A)>

The amorphous polyester resin (A) for use according to the present disclosure is not particularly limited. Although various amorphous polyester resins can be used, a polyester resin including diol units having a bridged alicyclic skeleton or diol units having a cyclic acetal skeleton is preferable among them, in considering the low ability to adsorb a drug solution and the oxygen barrier properties.

The diol unit having a bridged alicyclic skeleton is preferably a unit derived from a compound represented by any of the formulae (1) to (3), more preferably a unit derived from a compound (tricyclodecane dimethanol) represented by the formula (1).

[Formula 12]

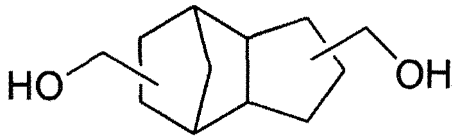

(1)

[Formula 13]

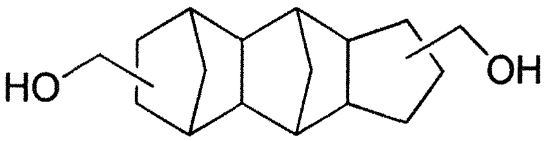

(2)

[Formula 14]

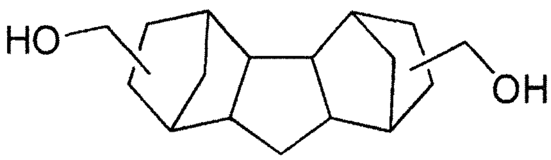

(3)

Specific examples of the compounds represented by the formulae (1) to (3) are the same as in the description of the polyester resin (A1). These may be used singly or in combinations of two or more at the same time.

The diol unit having a cyclic acetal skeleton according to the present disclosure is preferably the unit derived from a

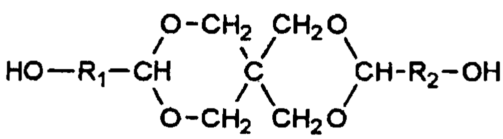

(4)

compound represented by the following formula (4) or (5).

[Formula 15]

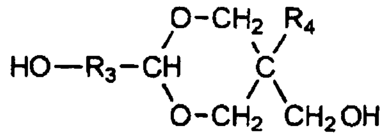

(5)

[Formula 16]

In Formulae (4) and (5), $R_1$ to $R_4$ each independently represent a hydrocarbon group selected from the group consisting of an aliphatic hydrocarbon group having 1 to 10 carbon atoms, an alicyclic hydrocarbon group having 3 to 10 carbon atoms, and an aromatic hydrocarbon group having 6 to 10 carbon atoms. Among the compounds represented by the Formulae (4) and (5), 3,9-bis(1,1-dimethyl-2-hydroxyethyl)-2,4,8,10-tetraoxaspiro[5.5]undecane, or 5-methylol-5-ethyl-2-(1,1-dimethyl-2-hydroxyethyl)-1,3-dioxane is particularly preferred.

The amorphous polyester resin (A) according to the present disclosure has a proportion of dicarboxylic acid units having a naphthalene skeleton in the total constituent dicarboxylic acid units of 70 mol% or more, in considering the high oxygen barrier properties and the high temperature resistance, as described in the following.

In an preferred aspect, the amorphous polyester resin (A) according to the present disclosure includes: a polyester resin (A1) of which diol units include at least one diol unit in an amount of 1 to 30 mol% selected from diol units having a bridged alicyclic skeleton derived from a compound represented by the formula (1), the formula (2), or the formula (3), and of which dicarboxylic acid units include dicarboxylic acid units having a naphthalene skeleton in an amount of 70 mol% or more; and a polyester resin (A2) of which diol units includes at least one diol unit in an amount of 1 to 30 mol% selected from diol units having an cyclic acetal skeleton derived from a compound represented by the formula (4) or the formula (5), and of which dicarboxylic acid units include dicarboxylic acid units having a naphthalene skeleton in an amount of 70 mol% or more.

According to the present disclosure, the proportion of diol units having a bridged alicyclic skeleton or a cyclic acetal skeleton in the total diol units of the amorphous polyester resin (A) is preferably 1 to 30 mol%. The amorphous polyester resin (A) including diol units having a bridged alicyclic skeleton or a cyclic acetal skeleton in an amount of 1 mol% or more has an increased glass transition temperature, so that high temperature resistance of the polyester resin is improved. In addition, since the crystallinity is reduced, partial crystallization during boiling disinfection and dimensional changes, whitening, and embrittlement associated therewith can be suppressed. The proportion of diol units having a bridged alicyclic skeleton or a cyclic acetal skeleton is preferably 3 mol% or more, more preferably 5 mol% or more. Meanwhile, a proportion of diol units having a bridged alicyclic skeleton or a cyclic acetal skeleton of more than 30 mol% in the total diol units of a polyester resin causes the reduction in the water vapor barrier properties and the oxygen barrier properties of the amorphous polyester resin (A), which may result in undesired consequences. In considering the high temperature resistance, the water vapor barrier properties, and the oxygen barrier properties of the amorphous polyester resin (A), the proportion of diol units having a bridged alicyclic skeleton or a cyclic acetal skeleton is preferably 1 to 25 mol%, more preferably 3 to 20 mol%, furthermore preferably 5 to 15 mol%.

The diol unit other than diol units having a bridged alicyclic skeleton or a cyclic acetal skeleton is not particularly limited, and the diol unit other than diol units having the bridged alicyclic skeleton among the diol units of the polyester resin (A1) may be used.

The proportion of the dicarboxylic acid units having a naphthalene skeleton in the entire dicarboxylic acid units of the amorphous polyester resin (A) is 70 mol% or more according to the present disclosure. The amorphous polyester resin (A) including the dicarboxylic acid units having a naphthalene skeleton in an amount of 70 mol% or more has an increased glass transition temperature, so that high temperature resistance, the water vapor barrier properties, and the oxygen barrier properties can be improved at the same time. On the other hand, the amorphous polyester resin (A) according to the present disclosure including the dicarboxylic acid units having a naphthalene skeleton in the entire dicarboxylic acid units of the polyester resin in an amount less than 70 mol% causes reduction in the water vapor barrier properties, the oxygen barrier properties and the high temperature resistance. In considering the high temperature resistance, the water vapor barrier properties, and the oxygen barrier properties of the amorphous polyester resin (A), the proportion of the carboxylic acid units having a naphthalene skeleton is or thus preferably 80 mol% or more, more preferably 90 mol% or more, furthermore preferably 95 mol% or more, particularly preferably 100 mol%.

The dicarboxylic acid unit having a naphthalene skeleton in the amorphous polyester resin (A) according to the present disclosure is not particularly limited, and the same dicarboxylic acid units having a naphthalene skeleton for use in the polyester resin (A1) can be used.

As in the case of the polyester resin (A1), the amorphous polyester resin (A) according to the present disclosure may include the dicarboxylic acid units other than the dicarboxylic units having a naphthalene skeleton. Such dicarboxylic acid units are not particularly limited, and the same dicarboxylic acid units other than the dicarboxylic acid units having a naphthalene skeleton for use in the polyester resin (A1) can be used.

As in the case of the polyester resin (A1), the amorphous polyester resin (A) according to the present disclosure may include units for the adjustment of melt viscoelasticity and molecular weight. Examples of the units include the same units to be contained in a polyester resin (A1) for the adjustment melt viscoelasticity and molecular weight.

In particular, in considering the high temperature resistance, the water vapor barrier properties, the oxygen barrier properties, and the mechanical performance, the amorphous polyester resin (A) according to the present disclosure preferably include: diol units having a bridged alicyclic skeleton or a cyclic acetal skeleton derived from at least one diol selected from the group consisting of tricyclo[5.2.1.$0^{2,6}$]decane-3,8-dimethanol, tricyclo[5.2.1.0 $^{2,6}$]decane-3,9-dimethanol, tricyclo[5.2.1.$0^{2,6}$]decane-4,8-dimethanol, and tricyclo[5.2.1.$0^{2,6}$]decane-4,9-dimethanol; diol units other than diol units having a bridged alicyclic skeleton or a cyclic acetal skeleton derived from ethylene glycol; and entire dicarboxylic acid units derived from 2,6-naphthalenedicarboxylic acid.

The glass transition temperature of the amorphous polyester resin (A) according to the present disclosure is not particularly limited. Based on the same reason as in the case of the polyester resin (A1), the value measured with a DSC is preferably 110°C or higher, more preferably 115°C or higher, furthermore preferably 120°C or higher. The upper limit of the glass transition temperature is not particularly limited, preferably 160°C or lower based on the same reason as in the case of the polyester resin (A1).

The water vapor barrier properties of the amorphous polyester resin (A) according to the present disclosure represented by the moisture permeability coefficient is not particularly limited. Based on the same reason as in the case of the polyester resin (A1), the moisture permeability coefficient is preferably 1 g·mm/$m^2$/day or less, more preferably 0.8 g·mm/$m^2$/day or less, furthermore preferably 0.6 g·mm/$m^2$/day or less.

The oxygen barrier properties of the amorphous polyester resin (A) according to the present disclosure represented by the oxygen permeability coefficient is not particularly limited. Based on the same reason as in the case of the polyester resin (A1), the oxygen permeability coefficient is preferably 10 cc·mm/$m^2$/day/atm or less, more preferably 5 cc·mm/$m^2$/day/atm or less, furthermore preferably 2 cc·mm/$m^2$/day/atm or less, particularly preferably 1.5 cc·mm/$m^2$/day/atm or less.

The melt viscosity of the amorphous polyester resin (A) according to the present disclosure represented by MFR described in JIS K 7210 is not particularly limited. Based on the same reason as in the case of the polyester resin (A1), the melt viscosity is preferably in the range of 1 to 40 g/10 minutes, more preferably 3 to 30 g/10 minutes, furthermore preferably 5 to 20 g/10 minutes.

As in the case of the polyester resin (A1), an antioxidant or the like may be added to the amorphous polyester resin (A) according to the present disclosure.

<Intermediate layer>

According to the present disclosure, the resin for constituting layer other than the layer A, i.e. an intermediate layer, is not particularly limited. Examples of the resin include a polyolefin resin, a polyamide resin, a polyester resin other than the amorphous polyester resin (A), and a saponified ethylene-vinyl acetate random copolymer.

An intermediate layer may contain two or more resins in the same layer, and may be composed of a single layer or a plurality of layers containing the same resin or different resins.

The thickness of the intermediate layer is not particularly limited, and appropriately determined depending on applications. In order to ensure various physical properties required for a medical packaging container, the thickness is preferably 20 to 7,000 μm, more preferably 50 to 6,000 μm, furthermore preferably 100 to 5,000 μm.

<Layer B>

In considering the high water vapor barrier properties, at least one layer of the intermediate layers of a multilayer packaging container in the present embodiment is preferably a layer (layer B) containing a polyolefin resin (B).

The layer B is preferably a layer which contains polyolefin resin (B) as a main component. Here, "as a main component" means that the amount of polyolefin resin (B) contained in the layer B is 70 mass% or more, preferably 80 mass% or more, more preferably 90 to 100 mass%. The layer B may include an additive or the like depending on the desired performance, in addition to the polyolefin resin (B).

The medical packaging container in the present embodiment may include a plurality of layers B, of which configuration may be the same or different from each other.

The thickness of the layer B is not particularly limited, and appropriately determined depending on the applications. In order to secure the various physical properties required for a medical packaging container, the thickness is preferably 20 to 7,000 μm, more preferably 50 to 6,000 μm, furthermore preferably 100 to 5,000 μm.

<Polyolefin resin (B)>

The polyolefin resin (B) of a multilayer packaging container in the present embodiment is not particularly limited. Specific examples of the resin (B) include a known resin such as polyethylene (low density polyethylene, medium density polyethylene, high density polyethylene, or straight-chain (linear) low density polyethylene), polypropylene, polybutene-1, poly-4-methylpentene-1, an ethylene-$\alpha$-olefin copolymer, a propylene-$\alpha$-olefin copolymer, an ethylene-$\alpha,\beta$-unsaturated carboxylic acid copolymer, and an ethylene-$\alpha,\beta$-unsaturated carboxylic acid ester copolymer. The preferred resins are a ring-opening polymer of cycloolefins such as norbornene and tetracyclododecene, or a derivative thereof, and a hydrogenated product thereof; and a copolymer produced by polymerization of a cycloolefin such as norbornene and tetracyclododecene, or a derivative thereof, and ethylene or propylene, the copolymer having a molecular chain including a cyclopentyl residue or a substituted cyclopentyl residue. In the specification, the cycloolefin includes monocyclic and polycyclic cycloolefins. A thermoplastic norbornene resin or a thermoplastic tetracyclododecene resin is preferred. Examples of the thermoplastic norbornene resin include a ring-opening polymer of norbornene monomers, a hydrogenated product thereof; an addition polymer of norbornene monomers; and an addition polymer of norbornene monomers and olefins. Examples of the thermoplastic tetracyclododecene resin are described in Japanese Patent Laid-Open No.3-14882, Japanese Patent Laid-Open No.3-122137, and Japanese Patent Laid-Open No.4-63807.

In particular, a cycloolefin copolymer (COC), i.e. a copolymer made from norbornene and an olefin such as ethylene as raw material, and a copolymer made from tetracyclododecene and an olefin such as ethylene as raw material are preferred. A cycloolefin polymer (COP), i.e. a hydrogenated polymer of open-ring polymerized norbornene is also preferred. The COC and the COP are described in, for example, Japanese Patent Laid-Open No.5-300939 and Japanese Patent Laid-Open No.5-317411.

The COC is commercially available, for example, under the trade name "APEL" (registered trademark) made by Mitsui Chemicals. The COP is commercially available, for example, under the trade name "ZEONEX" (registered trademark) or "ZEONOR" made by Nippon Zeon, or "DAIKYO RESIN CZ" (registered trademark) made by Daikyo Seiko.

The COP and the COC are the most preferred material, having chemical resistance and the chemical properties such as heat resistance and light resistance as well as a polyolefin resin, and having physical properties such as mechanical properties, melting, flow properties, and dimensional accuracy as well as an amorphous resin.

<Optional layer>

A multi-layer packaging container in the present embodiment may include an optional layer depending on the desired performance, in addition to the layer A and the layer B. Examples of the optional layers include an adhesive layer.

In the case that the practical adhesion strength between adjacent two layers is not achieved in a multilayer packaging container in the present embodiment, an adhesive layer is preferably arranged between the two layers.

The adhesive layer preferably contains a thermoplastic resin having adhesion properties. Examples of the thermoplastic resin having adhesion properties include an acid-modified polyolefin resin prepared by modifying a polyolefin resin such as polyethylene and polypropylene with an unsaturated carboxylic acid such as acrylic acid, methacrylic acid, maleic acid, maleic anhydride, fumaric acid, and itaconic acid; and a thermoplastic polyester elastomer mainly composed of a polyester block copolymer. In considering the adhesive properties, the same type of resin as the amorphous polyester resin (A) for use in the layer A is preferably modified for use as the adhesive layer.

In order to secure the practical adhesion strength and fabricability, the thickness of the adhesive layer is preferably 2 to 100 $\mu$m, more preferably 5 to 90 $\mu$m, furthermore preferably 10 to 80 $\mu$m.

[Medical packaging container]

A medical packaging container in the present embodiment is manufactured by forming a resin such as a polyester resin into a desired container shape by molding means such as injection molding, extrusion molding, and compression molding (sheet molding and blow molding).

The shape of the packaging container is not particularly limited. For example, a vial, an ampoule, and a prefilled syringe may be formed.

<Vial>

As a medical packaging container in the present embodiment, the vial is not different from a commonly-used vial, including a bottle, a rubber plug, and a cap. The bottle is filled with a drug solution, covered with a rubber plug, and tightly wrapped with a cap thereon so as to form a hermetically sealed medical packaging container. Preferably the bottle part is made of a multi-layer structure including the innermost layer and the outermost layer as layer A mainly composed of polyester resin (A1) in the present embodiment, and at least one layer of the intermediate layers as layer B mainly

composed of polyolefin resin (B) in the present embodiment.

The method for forming the bottle part of a vial is not particularly limited. Examples of the manufacturing method include injection blow molding and extrusion blow molding. As an example, a method for forming the bottle part in the multi-layer structure by injection blow molding is shown in the following.

For example, using a molding machine having two or more injection machines and an injection mold, a material to form the layer A and a material to form the layer B are injected into a cavity through mold hot runners from respective injection cylinders, and thus a multi-layer molded body corresponding to the shape of the injection mold can be manufactured. Alternatively, a material to form the layer A is firstly injected from a cylinder, a material to form the layer B is then injected from another cylinder concurrently with the injection of the resin to form the layer A, and the material to form the layer A is then injected in a necessary amount, so that the cavity is filled therewith. A molded body having a three-layer structure A/B/A can be thus manufactured.

Alternatively, a material to form the layer A is firstly injected, a material to form the layer B is then injected alone, and the material to form the layer A is finally injected in a necessary amount, so that the cavity is filled therewith. A molded body having a five-layer structure A/B/A/B/A can be thus manufactured.

Furthermore, a material to form the layer A is firstly injected from an injection cylinder, a material to form the layer B1 is then injected from another injection cylinder concurrently with the injection of the resin to form the layer A, a resin to form the layer B2 is then concurrently injected with the injection of the resin to form the layer A and the layer B1, and the resin to form the layer A is then injected in a necessary amount, consequently, the cavity is filled therewith. A multi-layer molded body having a five-layer structure A/B1/B2/B1/A can be thus manufactured.

In injection blow molding, a multi-layer molded body obtained by the method is fitted in a final shape mold (blow mold) in a state that the molded body is kept heated to some extent. The molded body is inflated with blowing air so as to come in close contact with the mold, being cooled and solidified into a bottle shape.

<Ampoule>

An ampoule in the present embodiment is not different from a commonly-used ampoule, which is a small medical packaging container with a narrow neck part to be filled with a drug solution and hermetically sealed by melting the tip of the neck part. The method for forming the ampoule is not particularly limited. Examples of the manufacturing method include injection blow molding and extrusion blow molding.

<Prefilled syringe>

The prefilled syringe in the present embodiment is not different from a commonly-used prefilled syringe, including at least a barrel to be filled with a drug solution, a joint part for joining a syringe needle to one end of the barrel, and a plunger for extruding the drug solution at the point of use, so as to form a medical container. In the case of a multi-layer packaging container, the innermost layer and the outermost layer of the barrel according to the present disclosure may be layer A including an amorphous polyester resin (A) and at least one layer of the intermediate layers may be a layer B including a polyolefin resin (B).

The prefilled syringe in the present embodiment may use a packing for enhancement of the adhesion between the plunger and the barrel. Although an amorphous polyester resin (A) may be used as the packing, a rubber elastic material such as a butyl rubber, a isoprene rubber, and a thermoplastic elastomer is more preferred. In the case that the plunger is in no contact with contents due to the use of a packing, examples of the resin for use in the plunger other than the amorphous polyester resin (A) include polypropylene, polyethylene, polycarbonate, and a cycloolefin polymer. In the case that the plunger is in contact with contents, the use of the amorphous polyester resin (A) in the plunger is preferred.

The method for forming the prefilled syringe in the present embodiment is not particularly limited, including, for example, a manufacturing method by injection molding.

The barrel of a multi-layer molded body is manufactured firstly by injecting a certain amount of resin to form a layer A into a cavity, subsequently injecting a certain amount of resin to form a layer B, and injecting a certain amount of resin to form a layer A once again. The barrel and the joint part may be integrally molded, or may be separately molded to be joined. The tip of the joint part is required to be sealed. Examples of the sealing method include heating the resin at the tip of the joint to a molten state and nipping with pliers to be fusion bonded.

The thickness of the container may be about 0.5 mm to about 20 mm, depending on the intended use and the size. The thickness may be either uniform or varied. In order to secure the long-term storage stability, another gas barrier film or a light blocking film may be formed on the (untreated) surface. Such a film and film forming method described in Japanese Patent Laid-Open No. 2004-323058 may be employed.

The filling material of the medical packaging container in the present embodiment is not particularly limited. In considering the effect of the present invention, for example, lipophilic compounds are preferred. In considering the

usability of the compounds, for example, terpenes and proteins are preferred. More specifically, preferred examples of the terpenes include lipophilic vitamins such as vitamin A, vitamin D, vitamin E, and vitamin K; monoterpene such as limonene, menthol, myrcene, ocimene, and cosmene; sesquiterpene such as farnesol, nerolidol, β-Shinensal, and caryophyllene; diterpene; sesterterpene; triterpene; and tetraterpene. Examples of the proteins include albumin such as egg albumin, serum albumin, and milk albumin. Terpenes modified with a compound having a peptide bond are also preferred as a filling material, including, for example, paclitaxel.

In the case that a medical packaging container in the present embodiment is filled with these compounds, the adsorption amount of the compounds is reduced and the deterioration due to oxidation and the dissipation of water content can be prevented.

Furthermore, before or after filling with the object to be stored, the medical packaging container and the object to be stored can be sterilized by a method suitable for the object to be stored. Examples of the sterilization method include thermal sterilization such as hydrothermal treatment at 100°C or lower, highpressure hydrothermal treatment at 100°C or higher, and high-temperature heating treatment at 121°C or higher; sterilization by electromagnetic waves such as ultraviolet, micro waves, and gamma rays; treatment by gas such as ethylene oxide; and sterilization by chemicals such as hydrogen per oxide and hypochlorous acid.

[Manufacturing method of polyester]

The manufacturing method of the polyester resin (A) in the present embodiment is not particularly limited. Any of known manufacturing methods of polyester may be applicable. Examples of the method include a melt polymerization method such as transesterification and direct esterification, and a solution polymerization method. In considering the ease of availability of raw materials, a transesterification method is preferred in the manufacturing methods of the polyester resin.

Any of various known catalysts such as transesterification catalysts, esterification catalysts, and polycondensation catalysts used in the manufacturing of the polyester resin, various known stabilizers such as etherification inhibitors, thermal stabilizers, and light stabilizers, and known polymerization modifiers can be used. These may be appropriately selected depending on the color tone of the polyester resin, the safety, the thermal stability, the weather resistance, and the solubility of themselves. Examples of the various catalysts include compounds of metal such as zinc, lead, cerium, cadmium, manganese, cobalt, lithium, sodium, potassium, calcium, nickel, magnesium, vanadium, aluminum, titanium, antimony, and tin (e.g., fatty acid salts, carbonates, phosphates, hydroxides, chlorides, oxides, and alkoxides), and metal magnesium. These may be used singly or in combinations of two or more. As a transesterification catalyst in a transesterification method, a compound of manganese is preferred among them, due to the high activity and less side reactions. As a polycondensation catalyst, a compound of antimony or titanium is preferred among them.

Preferably, the polyester resin (A) in the present embodiment is manufactured by a manufacturing method including the steps of producing an oligomer by a transesterification reaction of a dicarboxylic acid diester component containing naphthalenedicarboxylic acid diester and a diol component containing ethylene glycol and tricyclodecane dimethanol and/or pentacyclopentadecane dimethanol, i.e. at least one compound represented by any of the formulae (1) to (3) in the presence of a manganese compound, and polycondensing the oligomer in the presence of an antimony compound, with use of a phosphorus compound as a thermal stabilizer. The polyester resin has a manganese atom content of 40 to 200 ppm and an antimony atom content of 50 to 200 ppm, with a ratio (M/P) of the total content (M) (ppm) of manganese atoms and antimony atoms to the phosphorus atom content (P) (ppm) of 1.5 to 4.0.

In the manufacturing method in the present embodiment, the naphthalenedicarboxylic acid diester is a condensate of naphthalenedicarboxylic acid and alcohol. The number of carbon atoms of the alcohol is not particularly limited, preferably 1 to 6, more preferably 1 to 3, furthermore preferably 1 or 2, particularly preferably 1.

Specific examples of the naphthalenedicarboxylic acid diester include dimethyl 1,3-naphthalenedicarboxylate, diethyl 1,3-naphthalenedicarboxylate, dimethyl 1,4-naphthalenedicarboxylate, diethyl 1,4-naphthalenedicarboxylate, dimethyl 1,5-naphthalenedicarboxylate, diethyl 1,5-naphthalenedicarboxylate, dimethyl 2,6-naphthalenedicarboxylate, diethyl 2,6-naphthalenedicarboxylate, dimethyl 2,7-naphthalenedicarboxylate, and diethyl 2,7-naphthalenedicarboxylate. The dicarboxylic acid units having a naphthalene skeleton may be used singly or in combinations of two or more. In considering the high temperature resistance, the gas barrier properties, and the economic efficiency, dimethyl 2,6-naphthalenedicarboxylate is most preferred among those described above.

In the manufacturing method in the present embodiment, although the entire dicarboxylic acid diester components are preferably composed of naphthalenedicarboxylic acid diester components, other dicarboxylic acid diester components may be used within a range not impairing the objects of the present invention such as the transparency, the heat resistance, the low ability to adsorb contents, and the color tones. Examples of the other dicarboxylic acid diester components include: aromatic dicarboxylic acid diesters such as dimethyl terephthalate, diethyl terephthalate, dimethyl isophthalate, and isophthalic acid diethyl; aliphatic dicarboxylic acid diesters such as dimethyl glutarate, diethyl glutarate, dimethyl adipate, diethyl adipate, dimethyl sebacate, diethyl sebacate, dimethyl decanedicarboxylate, diethyl

decanedicarboxylate, dimethyl dodecanedicarboxylate, and diethyl dodecanedicarboxylate; and alicyclic dicarboxylic acid diesters such as dimethyl 1,3-cyclohexanedicarboxylate, diethyl 1,3-cyclohexanedicarboxylate, dimethyl 1,4-cyclohexanedicarboxylate, and diethyl 1,4-cyclohexanedicarboxylate. The dicarboxylic acid diester components may be used singly or in combinations of two or more.

In considering the adjustment of molecular weight, a dicarboxylic acid monoester component may be used with the dicarboxylic acid diester component.

In the manufacturing method in the present embodiment, the diol components include ethylene glycol and TCDDM and/or PCPDM. The ratio of ethylene glycol to the total of TCDDM and PCPDM is preferably 70 to 99 mol%/30 to 1 mol%, more preferably 80 to 95 mol%/20 to 5 mol%, furthermore preferably 90 to 95 mol%/10 to 5 mol%, for the total of ethylene glycol, TCDDM and PCPDM set to 100 mol%. A ratio of ethylene glycol to TCDDM and/or PCPDM in the range allows for further improvement in the heat resistance, the transparency, and the low ability to adsorb contents of a produced polyester resin.

In considering the low ability to adsorb a drug solution, the high temperature resistance, and the moldability, the TCDDM and/or PCPDM for use in the manufacturing method in the present embodiment is preferably selected from the group consisting of tricyclodecane dimethanol represented by the formula (1), pentacyclopentadecane dimethanol represented by the formula (2), and pentacyclopentadecane dimethanol represented by the formula (3).

In the manufacturing method in the present embodiment, the specific examples of the formulae (1) to (3) are the same as in the case of polyester resin (A1).

In the manufacturing method in the present embodiment, although the entire diol components are preferably composed of ethylene glycol and TCDDM and/or PCPDM, other diol components may be used within a range not impairing the objects of the present invention such as the transparency, the heat resistance, the low ability to adsorb contents, and the color tones.

Examples of the other diol components include units derived from aliphatic diols, cycloaliphatic diols, polyether compounds, bisphenols and alkylene oxide adducts thereof, and aromatic dihydroxy compounds and alkylene oxide adducts thereof.

Examples of the aliphatic diols include trimethylene glycol, 1,4-butanediol, 1,5-pentanediol, 1,6-hexanediol, diethylene glycol, propylene glycol, and neopentyl glycol.

Examples of the alicyclic diols include 1,3-cyclohexane dimethanol, 1,4-cyclohexane dimethanol, 1,2-decahydronaphthalene dimethanol, 1,3-decahydronaphthalene dimethanol, 1,4-decahydronaphthalene dimethanol, 1,5-decahydronaphthalene dimethanol, 1,6-decahydronaphthalene dimethanol, 2,7-decahydronaphthalene dimethanol, and tetralin dimethanol.

Examples of the polyether compounds include polyethylene glycol, polypropylene glycol, and polybutylene glycol.

Examples of the bisphenols include 4,4'-(1-methylethylidene)bisphenol, methylene bisphenol (bisphenol F), 4,4'-cyclohexylidene bisphenol (bisphenol Z), and 4,4'-sulfonyl bisphenol (bisphenol S).

Examples of the aromatic dihydroxy compound include xylylene glycol, hydroquinone, resorcin, 4,4'-dihydroxybiphenyl, 4,4'-dihydroxy diphenyl ether, and 4,4'-dihydroxy diphenyl benzophenone.

The diol components may be used singly or in combinations of two or more.

The manufacturing method in the present embodiment includes the steps of reacting the dicarboxylic acid diester component with the diol component by a transesterification reaction and a subsequent polycondensation reaction. More specifically, a polyester resin is manufactured by producing an oligomer by a transesterification reaction using a manganese compound as transesterification catalyst and then polycondensing the oligomer using an antimony compound as polycondensation catalyst.

In the manufacturing method in the present embodiment, a manganese compound is used as transesterification catalyst. In order to preserve the sufficient catalytic activity and prevent the polyester resin from becoming dark in color and yellowish in color, the manganese compound contained in the polyester resin has a manganese atom content of preferably 40 to 200 ppm, more preferably 45 to 150 ppm, furthermore preferably 50 to 100 ppm. A manganese atom content in the polyester resin of 40 ppm or more allow for the sufficient activity of transesterification. On the other hand, a manganese atom content in the polyester resin less than 200 ppm prevents the polyester resin from becoming dark in color and yellowish in color.

The manganese compound is not particularly limited, including, for example, an organic salt such as manganese acetate and manganese benzoate, a chloride such as manganese chloride, an alkoxide such as manganese methoxide, and manganese acetylacetonate. Among these, manganese acetate is preferably used in considering economic efficiency and availability.

In the manufacturing method in the present embodiment, the transesterification method is not particularly limited. Either of a batch process and a continuous process may be employed. The transesterification conditions in manufacturing normal polyester can be applicable. For example, the reaction temperature is preferably in the range of 120 to 250 °C, particularly preferably 140 to 220°C. Further, the reaction rate of the oligomer after transesterification is preferably 90%

or more.

The oligomer produced by the transesterification is then polycondensed.

In the manufacturing method in the present embodiment, an antimony compound is used as polycondensation catalyst. In order to preserve the sufficient polycondensation activity and prevent the polyester resin from becoming dark in color and yellowish in color, the antimony compound contained in the polyester resin has an antimony atom content of 50 to 200 ppm, preferably 60 to 150 ppm, more preferably 70 to 100 ppm. An antimony atom content for use in the polyester resin of 50 ppm or more allows for the sufficient activity of polycondensation. On the other hand, an antimony atom content for use in the polyester resin of 200 ppm or less prevents the polyester resin from becoming dark in color and yellowish in color.

The antimony compound is not particularly limited, including, for example, antimony trioxide, antimony pentoxide, and antimony acetate. Among these, in considering economic efficiency and availability, antimony trioxide is preferably used.

In the manufacturing method in the present embodiment, the polycondensation method is not particularly limited. Either of a batch process and a continuous process may be employed. The polycondensation conditions in manufacturing a normal polyester can be directly applicable. For example, the reaction temperature is preferably in the range of 240 to 285 °C, particularly preferably 255 to 275°C.

In the manufacturing method in the present embodiment, a phosphorus compound is used as a thermal stabilizer.

The timing of addition of the phosphorus compound is not particularly limited, preferably after the completion of transesterification and before the initiation of polycondensation.

In the manufacturing method in the present embodiment, the amount of the phosphorus compound for use is determined by the total content of the manganese compound and the antimony compound in the polyester resin. The phosphorus compound is contained in the polyester resin such that the ratio (M/P) of the total content (M) (ppm) of antimony atoms and manganese atoms (antimony atom content + manganese atom content) to the phosphorus atom content (P) (ppm) amounts to 1.5 to 4.0, preferably 2.0 to 3.5, more preferably 2.3 to 3.0. A content ratio (M/P) of 1.5 or more allows for sufficient polycondensation activity and effectively prevents the polyester resin from becoming dark in color. On the other hand, a content ratio (M/P) of 4.0 or less prevents the increase of yellowness of the polyester resin.

The phosphorus compound for use in the manufacturing method in the present embodiment is not particularly limited, and a known phosphorus compound may be used.

Examples of the phosphorus compound include phosphoric acid, phosphorous acid, phosphoric acid ester, and phosphorous acid ester. Examples of the phosphoric acid ester include methyl phosphate, ethyl phosphate, butyl phosphate, phenyl phosphate, dimethyl phosphate, diethyl phosphate, dibutyl phosphate, diphenyl phosphate, trimethyl phosphate, triethyl phosphate, tributyl phosphate, and triphenyl phosphate. Examples of the phosphorous acid ester include methyl phosphite, ethyl phosphite, butyl phosphite, phenyl phosphite, dimethyl phosphite, diethyl phosphite, dibutyl phosphite, diphenyl phosphite, trimethyl phosphite, triethyl phosphite, tributyl phosphite, and triphenyl phosphite.

Among them, phosphoric acid is particularly preferred, allowing phosphorus atoms to be quantitatively contained in the polyester resin. These may be used singly or in combinations of two or more.

In manufacturing the polyester resin, various kinds of stabilizers such as an etherification inhibitor, a thermal stabilizer, and a light stabilizer, and a polymerization modifier may be used.

Various kinds of additives and molding auxiliary agents such as an antioxidant, a light stabilizers, an ultraviolet absorber, a plasticizer, a bulking agent, a matting agent, a drying control agent, an antistatic agent, an antisettling agent, a surfactant, a flow modifier, a drying oil, waxes, a filler, a colorant, a reinforcing agent, a surface lubricant, a leveling agent, a curing accelerator, and a thickener may be added to the polyester resin within a range not impairing the object of the present invention.

Further, a colorant may be added to the produced polyester resin so as to reduce yellowness YI. The colorant may be added in the manufacturing of the polyester resin. The colorant is not particularly limited, and a known organic pigment or a known inorganic pigment may be used. Examples of the organic pigment include phthalocyanine and phthalo blue. Examples of the inorganic pigment include sodium aluminum sulfosilicate complex (A synthetic pigment composed of complex sodium aluminum sulfosilicate) and copper carbonate hydroxide. Among these, sodium aluminum sulfosilicate complex is particularly preferred in considering the safety and health.

In considering the reduction of yellowness YI of the produced polyester resin and the balance between the yellowness YI and the brightness L of the polyester resin, the amount of the colorant added in the polyester resin is preferably 1 to 100 ppm, more preferably 5 to 75 ppm, furthermore preferably 10 to 50 ppm.

The timing of addition of the colorant is not particularly limited, preferably during preparation of raw materials or after completion of transesterification and before initiation of polycondensation, in considering the dispersion of the colorant.

Examples

The following Examples are provided to further illustrate the present invention in more detail but are not intended to limit the scope of the invention. The evaluation methods are as follows.

<Evaluation of polyester resin>

(1) Resin composition

The proportions of the diol units and the dicarboxylic acid units in a polyester resin were calculated by $^1$H-NMR measurement. A nuclear magnetic resonance apparatus (trade name: JNM-AL400, made by JEOL Ltd.) was used at 400 MHz as the measurement apparatus. Deuterated trifluoroacetic acid/ deuterated chloroform (mass ratio: 1/9) was used as the solvent.

(2) Color tone

The color tone (brightness L and yellowness YI) of a polyester resin was measured for a pellet sample of the polyester resin by a reflection method in accordance with JIS K 7103. A color difference meter (trade name: COLOR METER ZE-2000, made by Nippon Denshoku Industries Co., Ltd.) was used as the measurement apparatus.

(3) Concentration of each atom

The concentration of each atom of a polyester resin was measured with an fluorescence X-ray spectrometer (trade name: ZSX Primus, made by Rigaku Corporation).

Based on the measured content of antimony atoms, manganese atoms and phosphorus atoms, the ratio (M/P) of the total content (M) of antimony atoms and manganese atoms to the content of phosphorus atoms was calculated.

(4) Glass transition temperature (Tg)

The glass transition temperature of a polyester resin was measured with a DSC/TA-60WS made by Shimadzu Corporation. Approximately 10 mg of polyester resin was placed in an unsealed container made of aluminum so as to be heated to 280°C in a nitrogen gas (30 ml/min) stream at a temperature increase rate of 20°C/min. The melted resin was quenched to form a measurement sample. The sample was measured under the same conditions, and the temperature corresponding to the changes by 1/2 of the difference between the baselines before and after the transition in the DSC curve was defined as the glass transition temperature.

(5) Melt flow rate (MFR)

The measurement was performed by a method in accordance with JIS K 7210, under conditions of 260°C and 2.16 kgf.

(6) Water vapor barrier properties

The water vapor barrier properties were evaluated by the moisture permeability coefficient. The moisture permeability coefficient was calculated from the following expression based on the water vapor permeation rate of a film having a thickness of 200 μm formed by melt extrusion as a measurement sample, which was measured with a LYSSY moisture permeation analyzer L80-4005L under measurement conditions at 40°C and 90% RH:

$$\text{Moisture permeability coefficient } (\text{g}\cdot\text{mm/m}^2\text{/day}) = \text{Water vapor permeation rate } (\text{g/m}^2\text{/day}) \times \text{Thickness (mm)}$$

(7) Oxygen barrier properties

The oxygen barrier properties were evaluated by the oxygen permeability coefficient. The oxygen permeability coefficient was calculated from the following expression based on the oxygen permeation rate of a film having a thickness of 200 μm formed by melt extrusion as a measurement sample, which was measured with a MOCON OX-TRAN2/21

under measurement conditions at 23°C and 65% RH:

$$\text{Oxygen permeability coefficient } (cc \cdot mm/m^2/day/atm) = \text{Oxygen permeation rate } (cc/m^2/day/atm) \times \text{Thickness } (mm)$$

(8) Ability to adsorb drug solution

The ability to adsorb a drug solution were evaluated by the increase amount in nitrogen concentration in a protein adsorption test.

The increase amount in nitrogen concentration was calculated from the following expression based on the nitrogen concentration of a film having a thickness of 100 μm formed by melt extrusion, which was immersed in an aqueous solution of protein for 8 days at 23°C and 50% RH and washed with pure water 5 times after immersion for the determination by nitrogen element analysis, and the nitrogen concentration of a film before immersion, which was preliminarily measured before filling with the aqueous solution of protein:

$$\text{Increase amount in nitrogen concentration (ppm)} = (\text{Nitrogen concentration of film after immersion (ppm)}) - (\text{Nitrogen concentration of film before immersion (ppm)})$$

As the aqueous solution of protein, a 1 wt% aqueous solution of albumin (bovine-derived powder) made by Sigma-Aldrich Japan KK was used. A total nitrogen analyzer TN-10 made by Mitsubishi Chemical Corporation was used for elemental analysis.

(9) Radiation sterilization resistance

The radiation sterilization resistance was evaluated by the difference ΔYI in the yellowness YI before and after irradiation with electron beams or gamma rays.

The yellowness (YI) of a plate having a thickness of 2 mm formed by melt extrusion as a measurement sample was measured using color difference instrument COH-300A manufactured by Nippon Denshoku Industries Co., Ltd to obtain the difference ΔYI between the yellowness (YI) of the plate measured 48 hours after forming and the yellowness (YI) of the plate measured immediately after irradiation with electron beams at 50 kGy or gamma rays at 50 kGy.

<Evaluation method of syringe>

(10) Mechanical strength

The mechanical strength was evaluated by a drop test.

A prefilled syringe filled with water was freely fallen from a height of 1.5 m, 5 times continuously. When none of the 10 samples tested broke, the strength was evaluated as passed.

(11) High temperature resistance

The high temperature resistance was evaluated by a boiling test.

After boiling sterilization of a prefilled syringe filled with water in boiling water for 10 minutes, the leakage between a plunger and a barrel and the change in appearance such as whitening were inspected. When none of both was inspected, the resistance was evaluated as passed.

<Evaluation method of vial>

(12) Oxygen barrier properties

The oxygen barrier properties were evaluated by oxygen permeation rate.

The oxygen permeation rate of a vial was measured after 15 days from the measurement initiation in accordance with ASTM D 3985 under the atmosphere at 23°C, with a relative humidity of 50% outside the molded body and a relative humidity of 100% inside the molded body. An oxygen permeation rate measurement apparatus (manufactured by MOCON Inc., trade name: OX-TRAN 2-21 ML) was used in the measurement. The lower the oxygen permeation rate is, the better the oxygen barrier properties are.

(13) Water vapor barrier properties

The water vapor barrier properties were evaluated by the water vapor permeation rate.

The water vapor permeation rate of a vial was measured after 15 days from the measurement initiation in accordance with ASTM D 3985 under the atmosphere at 23°C, with a relative humidity of 10% outside the molded body and a relative humidity of 100% inside the molded body.

A water vapor permeation rate measurement apparatus (manufactured by MOCON Inc., trade name: PERMATRAN 3/33MG) was used in the measurement. The lower the water vapor permeation rate is, the better the water vapor barrier properties are.

(14) Ability to adsorb drug solution

The ability to adsorb a drug solution were evaluated by the residual rate of protein.

A produced vial was filled with 100 cc of an aqueous solution of protein, which was then let stand for 30 days at 30°C and 100% RH. The nitrogen concentration of the aqueous solution was then measured by nitrogen analysis, so that the residual rate of protein in the aqueous solution was calculated from the following expression based on the nitrogen concentration:

Residual rate of protein in aqueous solution (%) = (Nitrogen concentration in aqueous solution after elapse of time (ppm)) / (Nitrogen concentration in aqueous solution before elapse of time (ppm)) × 100

As the aqueous solution of protein, 300 ppm of an aqueous solution of γ-globulin (derived from human serum, powder) made by Wako Pure Chemical Industries was used. In the elemental analysis, a total nitrogen analyzer TN-5 made by Mitsubishi Chemical Analytech Co., Ltd was used.

(15) High temperature resistance

After high temperature water vapor sterilization of a produced vial at 121°C for 20 minutes, the following evaluations were performed.

(15-1) Ability to adsorb drug solution

The residual rate of protein in aqueous solution was measured for a vial after high temperature water vapor sterilization, by the same method as in (14).

(15-2) Change in appearance

After high temperature water vapor sterilization, the change in appearance such as the deformation and the whitening of the vial was inspected. When no change in appearance was inspected, the resistance was evaluated as passed.

(16) Radiation sterilization resistance

(16-1) Ability to adsorb drug solution

A produced vial was filled with 100 cc of an aqueous solution of protein and irradiated with γ-ray at 25 kGy. Subsequently the vial was let stand for 30 days at 30°C and 100% RH, and the nitrogen concentration of the aqueous solution was measured by nitrogen analysis. The residual rate of protein in the aqueous solution was calculated from the following expression based on the measurement:

Residual rate of protein in aqueous solution (%) = (Nitrogen concentration in aqueous solution after elapse of time (ppm))/(Nitrogen concentration in aqueous solution before elapse of time (ppm)) × 100

As the aqueous solution of protein, 300 ppm of an aqueous solution of γ-globulin (derived from human serum, powder) made by Wako Pure Chemical Industries was used. In the elemental analysis, a total nitrogen analyzer TN-5 made by Mitsubishi Chemical Analytech Co., Ltd was used.

(16-2) Change in appearance

A produced vial was irradiated with γ-ray at 25 kGy, and the change in appearance such as the deformation and the discoloration of the vial was then inspected. A vial having change hardly observed was evaluated as passed.

(16-3) Mechanical strength

A produced vial was filled with 100 cc of water and sealed with an aluminum lid. After irradiation with γ-ray at 25 kGy, the vial was freely fallen from a height of 3 m, 5 times continuously. When none of the 10 samples tested broke, the strength was evaluated as passed.

(17) Moldability

The presence or absence of whitening in the vicinity of the gate of a produced vial was confirmed by visual observation. When no whitening was observed, the moldability was evaluated as passed.

[Example 1]

<Manufacturing and evaluation of polyester resin>

To a 150 L polyester manufacturing apparatus having a packed column type rectifying column, a partial condenser, a total condenser, a cold trap, a stirrer, a heating device, and a nitrogen inlet pipe, raw material monomers described in Table 1 were fed. Under the presence of 0.0255 mol% manganese acetate tetrahydrate relative to dicarboxylic acid diester components, the temperature was raised to 215°C under nitrogen atmosphere for performing transesterification reaction. After the reaction conversion rate of the dicarboxylic acid diester components reached 90% or more, 0.009 mol% antimony trioxide and 0.07 mol% phosphoric acid relative to the dicarboxylic acid diester components were added. The temperature was gradually increased and the pressure was gradually reduced, so that polycondensation was finally performed at 275°C, under 0.1 kPa or below. When the melt viscosity reached a suitable value, the reaction was terminated to obtain a polyester resin.

[Example 2]

A polyester resin was obtained by the same way as in Example 1, except that 0.03 mol% manganese acetate tetrahydrate relative to dicarboxylic acid diester components was added.

[Example 3]

A polyester resin was obtained by the same way as in Example 1, except that 0.06 mol% phosphoric acid relative to dicarboxylic acid diester components was added.

[Example 4]

A polyester resin was obtained by the same way as in Example 1, except that the amount of phosphoric acid added was changed to 0.06 mol% relative to dicarboxylic acid diester components, and 25 ppm of sodium aluminum sulfosilicate complex relative to the theoretical yield of a polyester resin was added together with antimony trioxide and phosphoric acid.

[Comparative Example 1]

A polyester resin was obtained by the same way as in Example 1, except that 0.025 mol% antimony trioxide and 0.06 mol% phosphoric acid, relative to dicarboxylic acid diester components, were added.

[Comparative Example 2]

A polyester resin was obtained by the same way as in Example 1, except that 0.05 mol% manganese acetate tetrahydrate and 0.06 mol% phosphoric acid, relative to dicarboxylic acid diester components, were added.

The meanings of abbreviations in the table are as follows:

NDCM: dimethyl 2,6-naphthalenedicarboxylate

TCDDM: tricyclodecane dimethanol (made by Oxea Japan KK)

EG: ethylene glycol

[Table 1]

| | | Example 1 | Example 2 | Example 3 | Example 4 | Comparative Example 1 | Comparative Example 2 |
|---|---|---|---|---|---|---|---|
| Amount of monomer fed (mol) | | | | | | | |
| Dicarboxylic acid diester component | NDCM | 226.9 | 226.9 | 226.9 | 226.9 | 226.9 | 226.9 |
| Diol component | TCDDM | 22.7 | 22.7 | 22.7 | 22.7 | 227 | 22.7 |
| | EG | 385.7 | 385.7 | 385.7 | 385.7 | 385.7 | 385.7 |
| Amount of catalyst fed (mol%) | | | | | | | |
| Manganese acetate tetrahydrate | | 0.0255 | 0.03 | 0.0255 | 0.0255 | 0.0255 | 0.05 |
| Antimony trioxide | | 0.009 | 0.009 | 0.009 | 0.009 | 0.025 | 0.009 |
| Amount of thermal stabilizer fed (mol%) | | | | | | | |
| Phosphoric acid | | 0.07 | 0.07 | 0.06 | 0.06 | 0.06 | 0.06 |
| Atomic concentration (ppm) | | | | | | | |
| Manganese | | 54 | 63 | 55 | 56 | 55 | 110 |
| Antimony | | 77 | 75 | 76 | 76 | 221 | 75 |
| Phosphorus | | 56 | 55 | 44 | 45 | 45 | 45 |
| M/P | | 2.34 | 2.51 | 2.98 | 2.93 | 6.13 | 4.11 |

(continued)

| Atomic concentration (ppm) | | | | | | | | |
|---|---|---|---|---|---|---|---|---|
| Copolymer composition (mol%) | NDCM | 100 | 100 | 100 | 100 | 100 | 100 |
| | TCDDM | 9.5 | 9.5 | 9.4 | 9.4 | 9.6 | 9.4 |
| | EG | 90.5 | 90.5 | 90.6 | 90.6 | 90.4 | 90.6 |
| Pellet color tone | | | | | | | |
| Brightness L | | 56.6 | 55.0 | 58.2 | 55.2 | 51.5 | 52.0 |
| Yellowness YI | | -0.3 | 1.0 | 0.6 | -10.6 | 9.4 | 7.9 |

[Examples 5 to 7 and Comparative Examples 3 to 6]

<Manufacturing and evaluation of polyester resin>

To a 50 L (Example 5) or 150 L (Examples 6 and 7, and Comparative Examples 3 to 6) polyester manufacturing apparatus having a packed column type rectifying column, a partial condenser, a total condenser, a cold trap, a stirrer, a heating device, and a nitrogen inlet pipe, raw material monomers described in Table 2 and manganese acetate tetrahydrate as a catalyst were fed. The temperature was raised to 215°C under nitrogen atmosphere for performing transesterification reaction. After the reaction conversion rate of the dicarboxylic acid diester components reached 90% or more, antimony (III) oxide and the phosphorous compound described in Table 2 were added. The temperature was gradually increased and the pressure was gradually reduced, so that polycondensation was finally performed at 275 to 280°C, under 0.1 kPa or below.

The produced polyester resin was molded by melt extrusion at 240 to 260°C with a 25 mm single spindle extruder having a T-die so as to obtain films having a thickness of 200 μm and a thickness of 100 μm.

The produced polyester resin and films were evaluated as described above. The results are shown in Table 2.

The meanings of abbreviations in the table are as follows:

NDCM: dimethyl 2,6-naphthalenedicarboxylate
DMT: dimethyl terephthalate
EG: ethylene glycol
TCDDM: tricyclodecane dimethanol
SPG: spiroglycol

<Manufacturing and evaluation of prefilled syringe>

The thus produced polyester resin was injection molded at a temperature condition of 240 to 260°C with an injection molding machine having a mold clamping force of 100 ton, so that a barrel and plunger having an integrated joint part was formed. A packing made of butyl rubber was fixed to the tip of the plunger, so that an injection container having a capacity of 5 ml was prepared. The produced injection container was evaluated as described above. The results are shown in Table 2.

[Comparative Example 7]

An injection container was prepared and evaluated in the same way as in Example 5, except that the polyester resin in Example 5 was replaced with a polypropylene J-452HP made by Prime Polymer Co., Ltd. The evaluation results are shown in Table 3.

[Comparative Example 8]

A syringe container was prepared and evaluated in the same way as in Example 5, except that the polyester resin in Example 5 was replaced with a cycloolefin copolymer TOPAS6013 made by Ticona GmbH. The evaluation results are shown in Table 3.

[Table 2]

| | | Example 5 | Example 6 | Example 7 | Comparative Example 3 | Comparative Example 4 | Comparative Example 5 | Comparative Example 6 |
|---|---|---|---|---|---|---|---|---|
| Amount of monomer fed (mol) | | | | | | | | |
| Dicarboxylic acid component | NDCM | 70.41 | 215.6 | 233.0 | - | 218.5 | 193.6 | 187.5 |
| | DMT | - | - | - | 369.5 | - | - | - |
| Diol component | TCDDM | 7.04 | 43.1 | 11.6 | - | - | - | 93.7 |
| | EG | 119.69 | 344.9 | 407.7 | 591.2 | 393.3 | 329.0 | 243.7 |
| | SPG | - | - | - | - | - | 19.4 | - |
| Amount of catalyst and thermal stabilizer fed [mol% (relative to dicarboxylic acid)] | | | | | | | | |
| Manganese acetate tetrahydrate | | 0.03 | 0.0255 | 0.0255 | 0.03 | 0.03 | 0.03 | 0.0255 |
| Antimony trioxide | | 0.02 | 0.009 | 0.009 | 0.02 | 0.02 | 0.02 | 0.009 |
| Triethyl phosphate | | 0.06 | | | 0.06 | 0.06 | 0.06 | |
| Phosphoric acid | | | 0.06 | 0.06 | | | | 0.06 |
| Final polymerization temperature (°C) | | 280 | 275 | 275 | 280 | 280 | 280 | 275 |
| Evaluation result of polyester resin | | | | | | | | |
| Polymerization composition (mol%) | NDCM | 100 | 100 | 100 | 0 | 100 | 100 | 100 |
| | TCDDM | 10 | 20 | 5 | 0 | 0 | 0 | 50 |
| | SPG | 0 | 0 | 0 | 0 | 0 | 10 | 0 |
| Glass transition temperature (Tg) (°C) | | 124 | 124 | 123 | 84 | 124 | 127 | 128 |
| MFR (g/10 minutes) | | 21 | 15 | 13 | 20 | 15 | 20 | 20 |
| Moisture permeability coefficient (g·mm/$m^2$/day) | | 0.50 | 0.49 | 0.52 | 8 | 0.44 | 0.72 | 0.45 |
| Oxygen permeability coefficient (cc·mm/$m^2$/day/atm) | | 1.3 | 1.7 | 1.1 | 4 | 0.6 | 1.8 | 7.5 |
| Radiation sterilization resistance | | | | | | | | |
| ΔYI (50 kGy electron beam) | | 7 | 6 | 6 | 7 | 8 | 13 | 8 |

(continued)

| | Example 5 | Example 6 | Example 7 | Comparative Example 3 | Comparative Example 4 | Comparative Example 5 | Comparative Example 6 |
|---|---|---|---|---|---|---|---|
| ΔYI (50 kGy gamma ray) | 12 | 11 | 11 | 7 | 13 | 21 | 12 |
| Evaluation result of prefilled syringe | | | | | | | |
| Nitrogen increase amount (ppm) | 2.5 | 2.0 | 3.5 | 5.0 | 5.0 | 6.0 | 1.8 |
| Drop test | Passed | Passed | Passed | Passed | Passed | Passed | Passed |
| Boiling test | Passed | Passed | Passed | Whitened/Deformed | Whitened/Leakage | Passed | Passed |

[Table 3]

| | Comparative Example 7 | Comparative Example 8 |
|---|---|---|
| Evaluation result of resin | | |
| Glass transition temperature (Tg) (°C) | <-20 | 145 |
| Moisture permeability coefficient (g·mm/$m^2$/day) | 0.28 | 0.06 |
| Oxygen permeability coefficient (cc.mm/$m^2$/day/atm) | 75 | 40 |
| Evaluation result of prefilled syringe | | |
| Nitrogen increase amount (ppm) | 31 | 9 |
| Drop test | Passed | Passed |
| Boiling test | Passed | Passed |

[Manufacturing Examples 1 and 2] Manufacturing Example 2 is provided for reference only and is not part of the invention.

<Manufacturing and evaluation of polyester resin>

To a 50 L (Manufacturing Example 1) or 150 L (Manufacturing Examples 2) polyester manufacturing apparatus having a packed column type rectifying column, a partial condenser, a total condenser, a cold trap, a stirrer, a heating device, and a nitrogen inlet pipe, raw material monomers described in Table 4 were fed. Under the presence of 0.03 mol% manganese acetate tetrahydrate relative to dicarboxylic acid diester components, the temperature was raised to 215°C under nitrogen atmosphere for performing transesterification reaction. After the reaction conversion rate of the dicarboxylic acid diester components reached 90% or more, 0.02 mol% antimony (III) oxide and 0.06 mol% triethyl phosphate relative to the dicarboxylic acid diester components were added. The temperature was gradually increased and the pressure was gradually reduced, so that polycondensation was finally performed at 280°C, under 0.1 kPa or below.
The produced polyester resin was evaluated as described above. The results are shown in Table 4.
The meanings of abbreviations in the table are as follows:

NDCM: dimethyl 2,6-naphthalenedicarboxylate
EG: ethylene glycol
TCDDM: tricyclodecane dimethanol
SPG: spiroglycol

[Table 4]

| | | Manufacturing Example 1 | Manufacturing Example 2 |
|---|---|---|---|
| Amount of monomer fed (mol) | | | |
| Dicarboxylic acid component | NDCM | 70.41 | 193.6 |
| Diol component | TCDDM | 7.04 | - |
| | SPG | - | 19.4 |
| | EG | 119.69 | 329.0 |
| Evaluation result of polyester resin | | | |
| Polymerization composition (mol%) | NDCM | 100 | 100 |
| | TCDDM | 10 | 0 |
| | SPG | 0 | 10 |
| Glass transition temperature (Tg) (°C) | | 124 | 127 |
| MFR (g/10 minutes) | | 21 | 20 |

[Example 8]

<Manufacturing and evaluation of vial>

Under the following conditions, a material to form a layer A is firstly injected from a cylinder, a material to form a layer B is then injected from another cylinder concurrently with the injection of the resin to form the layer A, and the material to form the layer A is then injected in a necessary amount, so that the cavity was filled therewith. A injection molded body (20 g) having a three-layer structure A/B/A was thus obtained. The injection molded body was then cooled to a predetermined temperature and transferred to a blow mold so as to be blow molded. A vial (bottle part) was thus manufactured. The mass of the layer A was 30 mass% relative to the total mass of the produced vial.
As the resin for forming the layer B, a cycloolefin copolymer (trade name: TOPAS6013, made by Ticona GmbH) was used. As the resin for forming the layer A, the polyester resin manufactured in Manufacturing Example 1 was used.

(Shape of vial)

Length: 100 mm; outer diameter: 50 mm; and thickness: 2 mm. In manufacturing the vial, an integrated injection blow molding machine (Model: IBS 85, 4-cavity, made by UNILOY) was used.

(Molding conditions of vial)

Injection cylinder temperature for layer A: 300°C
Injection cylinder temperature for layer B: 300°C
Resin flow path temperature in injection mold: 300°C
Blow temperature: 150°C
Blow mold cooling water temperature: 15°C

[Example 9] is provided for reference only and is not part of the invention.
A vial was manufactured in the same way as in Example 8, except that the resin for forming the layer A was changed to the polyester resin manufactured in Manufacturing Example 2.

[Comparative Example 9]

A vial was manufactured in the same way as in Example 8, except that a cycloolefin copolymer (trade name: TOPAS6013, made by Ticona GmbH) was used as the resin for forming the layer A, and the polyester resin manufactured in Manufacturing Example 1 was used as the resin for forming the layer B.

[Example 10]

A single-layer vial having the same shape as in Example 8 was manufactured using the polyester resin manufactured in Manufacturing Example 1.

[Comparative Example 10]

A single-layer vial having the same shape as in Example 8 was manufactured using a cycloolefin copolymer (trade name: TOPAS6013, made by Ticona GmbH).
In Table 5, the evaluation results for the respective vials are shown.

[Table 5]

| | Example 8 | Example 9 | Comparative Example 9 | Example 10 | Comparative Example 10 |
|---|---|---|---|---|---|
| Layer structure | 3-layer | 3-layer | 3-layer | Single-layer | Single-layer |
| Oxygen permeation rate (μl/vial/day/0.21 atm) | 3.4 | 3.6 | 8.4 | 2.1 | 87.1 |

(continued)

| | | Example 8 | Example 9 | Comparative Example 9 | Example 10 | Comparative Example 10 |
|---|---|---|---|---|---|---|
| Water vapor permeation rate (mg/vial/day) | | 0.43 | 0.44 | 0.29 | 0.91 | 0.24 |
| Residual rate of protein (%) | | 97 | 96 | 87 | 96 | 86 |
| High temperature sterilization | Residual rate of protein (%) | 96 | 95 | 84 | 96 | 84 |
| | Change in appearance | Passed | Passed | Passed | Passed | Passed |
| Gamma ray sterilization | Residual rate of protein (%) | 97 | 96 | 85 | 97 | 85 |
| | Change in appearance | Slightly yellowing (Passed) | Slightly yellowing (Passed) | Slightly yellowing (Passed) | Slightly yellowing (Passed) | Slightly yellowing (Passed) |
| | Drop test | Passed | Passed | Passed | Passed | Passed |
| Moldability | | Passed | Passed | Passed | Passed | Passed |

Industrial Applicability

The medical packaging container of the present invention has excellent mechanical strength, high temperature resistance, water vapor barrier properties, and oxygen barrier properties, with low ability to adsorb a drug solution, being applicable to ampoules, vials, and prefilled syringes.

Furthermore, the polyester produced by the manufacturing method of the present invention can remedy the disadvantages of a conventional polyester of PEN copolymerized with TCDDM or the like such as becoming dark in color and becoming yellowish in color, while maintaining the transparency, the heat resistance, the low ability to adsorb contents. Thus, the polyester can be widely used as a material for a film, a sheet, and a container. Among them, the polyester is particularly suitable for a material for a film and a container.

## Claims

1. A medical packaging container comprising a polyester resin (A1) comprising diol units and dicarboxylic units, the diol units comprising at least one diol unit in an amount of 1 to 30 mol% selected from diol units having a bridged alicyclic skeleton derived from a compound represented by formula (1), formula (2), or formula (3), the dicarboxylic acid units comprising dicarboxylic acid units having a naphthalene skeleton in an amount of 70 mol% or more.

[Formula 1]

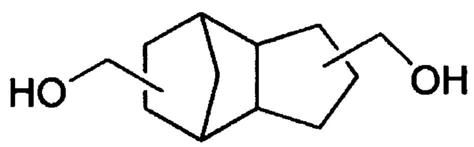

(1)

[Formula 2]

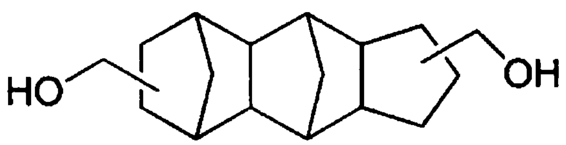

(2)

[Formula 3]

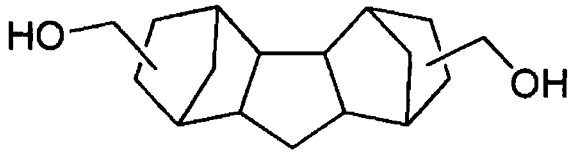

(3)

2. The medical packaging container according to claim 1, wherein the diol units comprise at least one diol unit in an amount of 1 to 30 mol% selected from diol units having a bridged alicyclic skeleton derived from a compound represented by the formula (1) or the formula (2).

3. The medical packaging container according to claim 1, wherein the diol units comprise diol units having a bridged alicyclic skeleton derived from a compound represented by the formula (1) in an amount of 1 to 30 mol%.

4. The medical packaging container according to any one of claims 1 to 3, wherein the dicarboxylic acid units having a naphthalene skeleton are derived from at least one dicarboxylic acid unit selected from the group consisting of 1,3-naphthalenedicarboxylic acid, 1,4-naphthalenedicarboxylic acid, 1,5-naphthalenedicarboxylic acid, 2,6-naphthalenedicarboxylic acid, and 2,7-naphthalenedicarboxylic acid.

5. The medical packaging container according to any one of claims 1 to 4, wherein the dicarboxylic acid units comprise the dicarboxylic acid units having a naphthalene skeleton in an amount of 90 mol% or more.

6. The medical packaging container according to any one of claims 1 to 5, wherein the diol units comprise diol units derived from ethylene glycol.

7. The medical packaging container according to any one of claims 1 to 6, wherein the polyester resin (A1) has all of the following properties (i) to (iii):

(i) a glass transition temperature of 110°C or higher as measured with a differential scanning calorimeter;
(ii) a moisture permeability coefficient of 1 gmm/$m^2$/day or less; and
(iii) an oxygen permeability coefficient of 10 ccmm/$m^2$/day/atm or less,

wherein the moisture permeability coefficient (g·mm/$m^2$/day) = water vapor permeation rate(g/$m^2$/day)×thickness (mm), based on the water vapor permeation rate of a film having a thickness of 200 μm formed by melt extrusion, under measurement conditions at 40°C and 90% RH;
wherein the water vapor permeation rate was measured in accordance with ASTM D 3985;
and the oxygen permeability coefficient (cc·mm/$m^2$/day/atm) = oxygen permeation rate (cc/$m^2$/day/atm)×thickness (mm) based on the oxygen permeation rate of a film having a thickness of 200 μm formed by melt extrusion, under measurement conditions at 23°C and 65% RH;
wherein the oxygen permeation rate was measured in accordance with ASTM D 3985.

8. The medical packaging container according to claim 7, wherein the polyester resin (A1) has the following property (iv) :

(iv) an increase amount in nitrogen concentration of 5 ppm or less when a polyester resin film having a thickness of 100 μm is immersed in a 1 wt.% aqueous solution of albumin, wherein the increase in nitrogen concentration (ppm)=(Nitrogen concentration of film after immersion (ppm))-(Nitrogen concentration of film before immersion (ppm)),

wherein the nitrogen concentration of film after immersion (ppm) is based on the nitrogen concentration of a film having a thickness of 100 μm produced by melt extrusion, which is immersed in an aqueous solution of protein for 8 days at 23°C and 50% RH and washed with pure water 5 times after immersion for the determination by nitrogen element analysis,
wherein the nitrogen concentration of film before immersion is preliminarily measured before filling with the aqueous solution of protein.

9. The medical packaging container according to any one of claims 1 to 8, wherein the polyester resin (A1) has the

following property (v):

(v) a melt flow rate in accordance with JIS K 7210 of 1 to 40 g/10 minutes under conditions of 260°C and 2.16 kgf.

**10.** The medical packaging container according to any one of claims 1 to 9, wherein the polyester resin (A1) has a manganese atom content of 40 to 200 ppm and an antimony atom content of 50 to 200 ppm, with a ratio (M/P) of the total content (M) (ppm) of manganese atoms and antimony atoms to the phosphorus atom content (P) (ppm) of 1.5 to 4.0.

**11.** The medical packaging container according to claim 10, wherein the polyester resin (A1) is obtained by a manufacturing method comprising the steps of:

producing an oligomer by transesterification of a dicarboxylic acid diester component comprising naphthalenedicarboxylic acid diester and a diol component comprising at least one selected from the compounds represented by the formula (1), the formula (2), and the formula (3) in the presence of a manganese compound; and polycondensing the oligomer in the presence of an antimony compound; and

wherein, in the manufacturing method, a phosphorus compound is used as a thermal stabilizer.

**12.** The medical packaging container according to claim 11, wherein the diol component further comprises ethylene glycol.

**13.** The medical packaging container according to any one of claims 1 to 12, comprising a multi-layer structure having at least three layers, and
an innermost layer and an outermost layer in the multi-layer structure comprising the polyester resin (A1).

**14.** The medical packaging container according to claim 13, wherein at least one layer of the intermediate layers in the multi-layer structure comprises a polyolefin resin (B).

**15.** The medical packaging container according to claim 14, wherein the polyolefin resin (B) is at least one selected from cycloolefin polymer, cycloolefin copolymer, and polypropylene.

**16.** The medical packaging container according to any one of claims 1 to 15, wherein the medical packaging container is an ampoule, a vial, or a prefilled syringe.

**17.** A manufacturing method of a polyester resin, comprising the steps of:

producing an oligomer by transesterification of a dicarboxylic acid diester component comprising naphthalenedicarboxylic acid diester and at least one selected from the compounds represented by formula (1), formula (2), and formula (3), and a diol component comprising ethylene glycol in the presence of a manganese compound; and
polycondensing the oligomer in the presence of an antimony compound;

wherein a phosphorus compound is used as a thermal stabilizer, and
wherein the polyester resin has a manganese atom content of 40 to 200 ppm and an antimony atom content of 50 to 200 ppm, with a ratio (M/P) of the total content (M) (ppm) of manganese atoms and antimony atoms to the phosphorus atom content (P) (ppm) of 1.5 to 4.0.

[Formula 6]

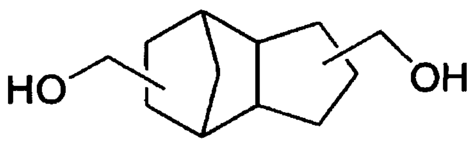

(1)

[Formula 7]

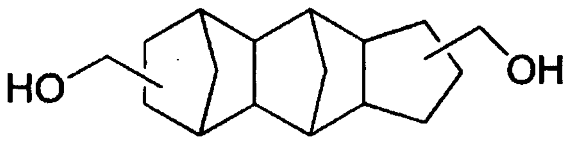

(2)

[Formula 8]

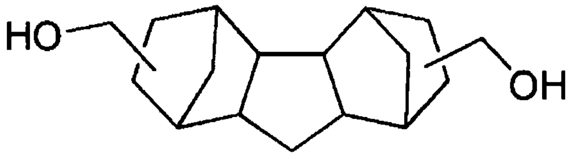

(3)

**18.** The manufacturing method of the polyester resin according to claim 17, wherein the manganese atoms are derived from manganese acetate, the antimony atoms are derived from antimony trioxide, and phosphorus atoms are derived from phosphoric acid.

**19.** The manufacturing method of the polyester resin according to claim 17 or 18, wherein the ratio of ethylene glycol to the total of the compounds represented by the formula (1), the formula (2), and the formula (3) in the diol components is 70 to 99 mol%/30 to 1 mol%.

**20.** The manufacturing method of the polyester resin according to any one of claims 17 to 19, wherein the naphthalenedicarboxylic acid diester is dimethyl 2,6-naphthalenedicarboxylate.

**Patentansprüche**

**1.** Medizinischer Verpackungsbehälter umfassend ein Polyesterharz (A1), welches Dioleinheiten und Dicarbonsäureeinheiten umfasst,
wobei die Dioleinheiten mindestens eine Dioleinheit in einer Menge von 1 bis 30 Mol% umfassen, die ausgewählt wird aus Dioleinheiten, die ein verbrücktes alicyclisches Gerüst aufweisen, das von einer Verbindung der Formel (1), der Formel (2), oder der Formel (3) abgeleitet ist,
wobei die Dicarbonsäureeinheiten Dicarbonsäureeinheiten, die ein Naphthalingerüst aufweisen, in einer Menge von 70 Mol% oder mehr umfassen.

[Formel 1]

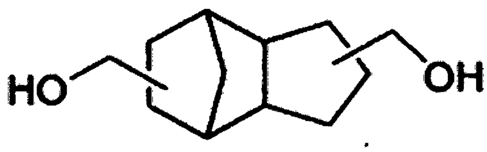

(1)

[Formel 2]

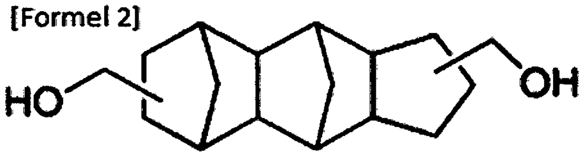

(2)

[Formel 3]

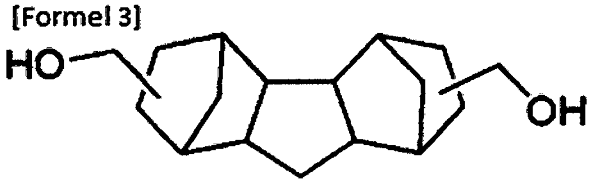

(3)

2. Medizinischer Verpackungsbehälter gemäß Anspruch 1, wobei die Dioleinheiten mindestens eine Dioleinheit in einer Menge von 1 bis 30 Mol% umfassen, die ausgewählt wird aus Dioleinheiten, die ein verbrücktes alicyclisches Gerüst aufweisen, das von der Formel (1), der Formel (2), oder der Formel (3) abgeleitet ist.

3. Medizinischer Verpackungsbehälter gemäß Anspruch 1, wobei die Dioleinheiten Dioleinheiten, die ein verbrücktes alicyclisches Gerüst aufweisen, das von der Formel (1) abgeleitet ist in einer Menge von 1 bis 30 Mol% umfassen.

4. Medizinischer Verpackungsbehälter gemäß einem der Ansprüche 1 bis 3, wobei die Dicarbonsäureeinheiten, die ein Naphthalingerüst aufweisen, abgeleitet sind von mindestens einer Dicarbonsäureeinheit ausgewählt aus der Gruppe bestehend aus 1,3-Naphthalindicarbonsäure, 1,4-Naphthalindicarbonsäure, 1,5-Naphthalindicarbonsäure, 2,6-Naphthalindicarbonsäure, und 2,7-Naphthalindicarbonsäure.

5. Medizinischer Verpackungsbehälter gemäß einem der Ansprüche 1 bis 4, wobei die Dicarbonsäureeinheiten Dicarbonsäureeinheiten, die ein Naphthalingerüst aufweisen in einer Menge von 90 Mol% oder mehr umfassen.

6. Medizinischer Verpackungsbehälter gemäß einem der Ansprüche 1 bis 5, wobei die Dioleinheiten von Ethylenglykol abgeleitete Dioleinheiten umfassen.

7. Medizinischer Verpackungsbehälter gemäß einem der Ansprüche 1 bis 6, wobei das Polyesterharz (A1) alle der folgenden Eigenschaften (i) bis (iii) aufweist:

   (i) eine Glasübergangstemperatur von 110°C oder mehr, bestimmt durch dynamische Differenzkalorimetrie (DSC);
   (ii) einen Feuchtigkeitsdurchlässigkeitskoeffizienten von 1 g·mm/m$^2$/Tag oder weniger; und
   (iii) einen Sauerstoffdurchlässigkeitskoeffizienten von 10 cc·mm/m$^2$/Tag/atm oder weniger,

   wobei der Feuchtigkeitsdurchlässigkeitskoeffizient (g·mm/m$^2$/Tag) = Wasserdampfdurchlässigkeitsrate (g/m$^2$/Tag) x Schichtdicke (mm) auf der Wasserdampfdurchlässigkeitsrate einer durch Schmelzextrusion gebildeten Folie mit einer Schichtdicke von 200 $\mu$m unter Messbedingungen von 40°C und 90% RH basiert,
   wobei die Wasserdampfdurchlässigkeitsrate entsprechend ASTM D 3985 gemessen wurde;
   und der Sauerstoffdurchlässigkeitskoeffizient (cc·mm/m$^2$/Tag/atm) = Sauerstoffdurchlässigkeitsrate (cc/m$^2$/Tag/atm) x Schichtdicke (mm) auf der Sauerstoffdurchlässigkeitsrate einer durch Schmelzextrusion gebildeten Folie mit einer Schichtdicke von 200 $\mu$m unter Messbedingungen von 23°C und 65% RH basiert;
   wobei die Sauerstoffdurchlässigkeitsrate entsprechend ASTM D 3985 gemessen wurde;

8. Medizinischer Verpackungsbehälter gemäß Anspruch 7, wobei das Polyesterharz (A1) die folgende Eigenschaft (iv) aufweist:

   (iv) einen Anstieg der Stickstoffkonzentration von 5 ppm oder weniger, wenn eine Polyesterharzfolie mit einer Schichtdicke von 100 $\mu$m in eine wässrige Lösung von 1 Gew.-% Albumin eingetaucht wird,

   wobei der Anstieg der Stickstoffkonzentration

   wobei der Anstieg der Stickstoffkonzentration (ppm) = (Stickstoffkonzentration der Folie nach dem Eintauchen (ppm)) − (Stickstoffkonzentration der Folie vor dem Eintauchen (ppm)),

   wobei die Stickstoffkonzentration der Folie nach dem Eintauchen (ppm) auf der Stickstoffkonzentration einer durch

Schmelzextraktion gebildeten Folie mit einer Schichtdicke von 100 μm basiert, welche für 8 Tage bei 23°C und 50% RH in eine wässrige Proteinlösung eingetaucht wurde und nach dem Eintauchen 5 Mal mit reinem Wasser für die Bestimmung mittels Stickstoffelementanalyse gewaschen wurde,
wobei die Stickstoffkonzentration der Folie vor dem Eintauchen vor der Befüllung mit der wässrigen Proteinlösung vorläufig gemessen wird.

**9.** Medizinischer Verpackungsbehälter gemäß einem der Ansprüche 1 bis 8, wobei das Polyesterharz (A1) die folgende Eigenschaft (v) aufweist:

(v) Eine Schmelzflussrate gemäß JIS K 7210 von 1 bis 40 g/10 Minuten bei 260°C und 2.16 kgf.

**10.** Medizinischer Verpackungsbehälter gemäß einem der Ansprüche 1 bis 9, wobei das Polyesterharz (A1) einen Manganatomgehalt von 40 bis 200 ppm und einen
Antimonatomgehalt von 50 bis 200 ppm aufweist, mit einem Verhältnis (M/P) des Gesamtgehalts (M) (ppm) an Manganatomen und Antimonatomen zu dem Phosphoratomgehalt (P) (ppm) von 1.5 bis 4.0.

**11.** Medizinischer Verpackungsbehälter gemäß Anspruch 10, wobei das Polyesterharz (A1) erhalten wird durch ein Herstellungsverfahren umfassend die Schritte:

Herstellen eines Oligomers durch Umesterung einer Dicarbonsäurediesterkomponente, die einen Naphthalindicarbonsäurediester umfasst, und einer Diolkomponente, die mindestens eine Verbindung umfasst, die ausgewählt wird aus den Verbindungen der Formel (1), der Formel (2) und der Formel (3) in der Gegenwart einer Manganverbindung; und
Polykondensieren des Oligomers in der Gegenwart einer Antimonverbindung; und
wobei im Herstellungsverfahren eine Phosphorverbindung als thermischer Stabilisator verwendet wird.

**12.** Medizinischer Verpackungsbehälter gemäß Anspruch 11, wobei die Diolkomponente ferner Ethylenglykol umfasst.

**13.** Medizinischer Verpackungsbehälter gemäß einem der Ansprüche 1 bis 12, umfassend eine mehrschichtige Struktur die mindestens drei Schichten aufweist, und wobei die
innerste Schicht und die äußerste Schicht in der mehrschichtigen Struktur das Polyesterharz (A1) umfassen.

**14.** Medizinischer Verpackungsbehälter gemäß Anspruch 13, wobei mindestens eine Schicht der Zwischenschichten in der mehrschichtigen Struktur ein Polyolefinharz (B) umfasst.

**15.** Medizinischer Verpackungsbehälter gemäß Anspruch 14, wobei das Polyolefinharz (B) mindestens eines ist, das ausgewählt wird aus Cycloolefinpolymer, Cycloolefincopolymer, und Polypropylen.

**16.** Medizinischer Verpackungsbehälter gemäß einem der Ansprüche 1 bis 15, wobei der medizinische Verpackungsbehälter eine Ampulle, ein Vial, oder eine vorgefüllte Spritze ist.

**17.** Herstellungsverfahren für ein Polyesterharz, welches die folgenden Schritte umfasst:

Herstellen eines Oligomers durch Umesterung einer Dicarbonsäurediesterkomponente, die einen Naphthalindicarbonsäurediester umfasst, und mindestens einer Verbindung, die ausgewählt wird aus den Verbindungen der Formel (1), der Formel (2), und der Formel (3), und einer Diolkomponente, die Ethylenglykol umfasst, in der Gegenwart einer Manganverbindung; und
Polykondensieren des Oligomers in der Gegenwart einer Antimonverbindung;

wobei eine Phosphorverbindung als thermischer Stabilisator verwendet wird, und
wobei das Polyesterharz einen Manganatomgehalt von 40 bis 200 ppm und einen Antimonatomgehalt von 50 bis 200 ppm aufweist, mit einem Verhältnis (M/P) des Gesamtgehalts (M) (ppm) an Manganatomen und Antimonatomen zu dem Phosphoratomgehalt (P) (ppm) von 1.5 bis 4.0.

[Formel 6]

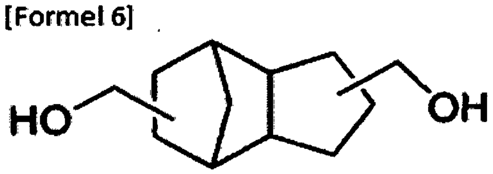

(1)

[Formel 7]

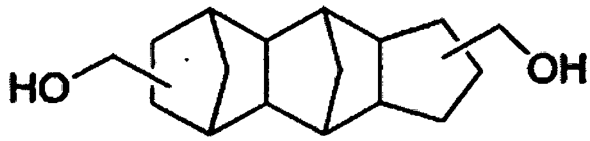

(2)

[Formel 8]

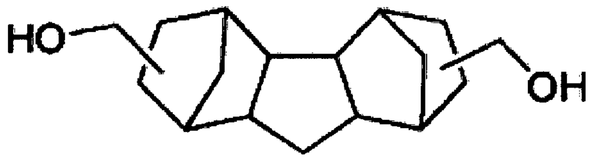

(3)

**18.** Herstellungsverfahren für ein Polyesterharz gemäß Anspruch 17, wobei die Manganatome von Manganacetat abgeleitet sind, die Antimonatome von Antimontrioxid abgeleitet sind, und die Phosphoratome von Phosphorsäure abgeleitet sind.

**19.** Herstellungsverfahren für ein Polyesterharz gemäß Anspruch 17 oder 18, wobei das Verhältnis von Ethylenglykol zur Gesamtmenge der Verbindungen der Formel (1), der Formel (2) und der Formel (3) in der Diolkomponente 70 bis 99 mol% / 30 bis 1 mol% beträgt.

**20.** Herstellungsverfahren für ein Polyesterharz gemäß einem der Ansprüche 17 bis 19, wobei der Naphthalindicarbonsäurediester Dimethyl-2,6-Naphthalindicarboxylat ist.

## Revendications

**1.** Récipient d'emballage médical comprenant une résine de polyester (A1) comprenant des unités diol et des unités dicarboxylique,
les unités diol comprenant au moins une unité diol en une quantité de 1 à 30 % en moles sélectionnée parmi des unités diol ayant un squelette alicyclique ponté dérivées d'un composé représenté par la formule (1), la formule (2), ou la formule (3),
les unités d'acide dicarboxylique comprenant des unités d'acide dicarboxylique ayant un squelette de naphtalène en une quantité de 70 % en moles ou plus.

[Formule 1]

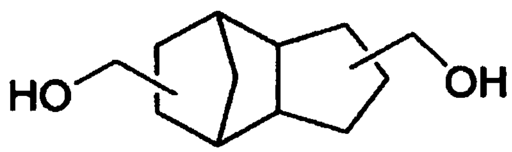

(1)

[Formule 2]

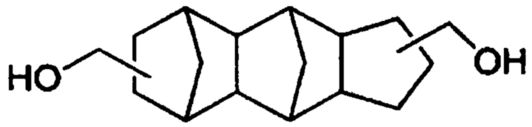

(2)

[Formule 3]

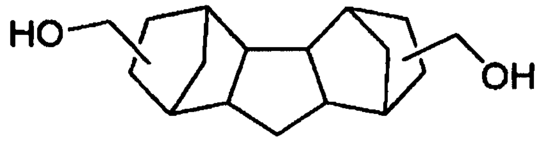

(3)

2. Récipient d'emballage médical selon la revendication 1, dans lequel les unités diol comprennent au moins une unité diol en une quantité de 1 à 30 % en moles sélectionnée parmi des unités diol ayant un squelette alicyclique ponté dérivé d'un composé représenté par la formule (1) ou la formule (2).

3. Récipient d'emballage médical selon la revendication 1, dans lequel les unités diol comprennent des unités diol ayant un squelette alicyclique ponté dérivé d'un composé représenté par la formule (1) en une quantité de 1 à 30 % en moles.

4. Récipient d'emballage médical selon l'une quelconque des revendications 1 à 3, dans lequel les unités d'acide dicarboxylique ayant un squelette de naphtalène sont dérivées d'au moins une unité d'acide dicarboxylique sélectionnée dans le groupe constitué de l'acide 1,3-naphthalènedicarboxylique, l'acide 1,4-naphthalènedicarboxylique, l'acide 1,5-naphthalènedicarboxylique, l'acide 2,6-naphthalènedicarboxylique, et l'acide 2,7-naphthalènedicarboxylique.

5. Récipient d'emballage médical selon l'une quelconque des revendications 1 à 4, dans lequel les unités d'acide dicarboxylique comprennent les unités d'acide dicarboxylique ayant un squelette de naphtalène en une quantité de 90 % en moles ou plus.

6. Récipient d'emballage médical selon l'une quelconque des revendications 1 à 5, dans lequel les unités diol comprennent des unités diol dérivées de l'éthylène glycol.

7. Récipient d'emballage médical selon l'une quelconque des revendications 1 à 6, dans lequel la résine de polyester (A1) a toutes les propriétés (i) à (iii) suivantes :

(i) une température de transition vitreuse de 110°C ou plus telle que mesurée avec un calorimètre à balayage différentiel ;
(ii) un coefficient de perméabilité à l'humidité de 1 g.mm/$m^2$/jour ou moins; et
(iii) un coefficient de perméabilité à l'oxygène de 10cc.mm/$m^2$/jour/atm ou moins,

dans lequel le coefficient de perméabilité à l'humidité (g.mm/$m^2$/jour) = taux de pénétration de vapeur d'eau (g/$m^2$/jour) x épaisseur (mm), sur la base du taux de pénétration de vapeur d'eau d'un film ayant une épaisseur de 200 $\mu$m formé par extrusion à l'état fondu, dans des conditions de mesure à 40°C et 90 % de HR ; dans lequel le taux de pénétration de vapeur d'eau a été mesuré selon ASTM D 3985 ;
et le coefficient de perméabilité à l'oxygène (cc.mm/$m^2$/jour/atm) = taux de pénétration d'oxygène (cc/$m^2$/jour/atm) x épaisseur (mm) sur la base du taux de pénétration d'oxygène d'un film ayant une épaisseur de 200 $\mu$m formé par extrusion à l'état fondu, dans des conditions de mesure à 23°C et 65 % de HR; dans lequel le taux de pénétration d'oxygène a été mesuré selon ASTM D 3985.

8. Récipient d'emballage médical selon la revendication 7, dans lequel la résine de polyester (A1) a la propriété (iv) suivante :

(iv) une quantité augmentée en concentration d'azote de 5 ppm ou moins lorsqu'un film de résine de polyester ayant une épaisseur de 100 $\mu$m est immergé dans une solution aqueuse à 1 % en poids d'albumine, dans

lequel l'augmentation en concentration d'azote

dans lequel l'augmentation en concentration d'azote (ppm) = (Concentration d'azote du film après immersion (ppm)) – (Concentration d'azote du film avant immersion (ppm)),

dans lequel la concentration d'azote du film après l'immersion (ppm) est basée sur la concentration d'azote d'un film ayant une épaisseur de 100 μm produit par extrusion à l'état fondu, qui est immergé dans une solution aqueuse de protéine pendant 8 jours à 23°C et 50 % de HR et lavé avec de l'eau pure 5 fois après l'immersion pour la détermination par analyse d'élément azote,
dans lequel la concentration d'azote du film avant l'immersion est mesurée préalablement au remplissage avec la solution aqueuse de protéine.

**9.** Récipient d'emballage médical selon l'une quelconque des revendications 1 à 8, dans lequel la résine de polyester (A1) a la propriété (v) suivante :

(v) un débit d'écoulement à l'état fondu selon JIS K 7210 de 1 à 40 g/10 minutes dans des conditions de 206°C et 2,16 kgf.

**10.** Récipient d'emballage médical selon l'une quelconque des revendications 1 à 9, dans lequel la résine de polyester (A1) a une teneur en atomes de manganèse de 40 à 200 ppm et un teneur en atomes d'antimoine de 50 à 200 ppm, avec un rapport (M/P) de la teneur total (M) (ppm) d'atomes de manganèse et d'atomes d'antimoine sur la teneur (P) (ppm) en atomes de phosphore de 1,5 à 4.

**11.** Récipient d'emballage médical selon la revendication 10, dans lequel la résine de polyester (A1) est obtenue par un procédé de fabrication comprenant les étapes de :

produire un oligomère par transestérification d'un composant diester d'acide dicarboxylique comprenant un diester d'acide naphthalènedicarboxylique et un composant diol comprenant au moins un sélectionné parmi les composés représentés par la formule (1), la formule (2), et la formule (3) en présence d'un composé de manganèse ; et
polycondenser l'oligomère en présence d'un composé d'antimoine ; et

dans lequel, dans le procédé de fabrication, un composé de phosphore est utilisé en tant que stabilisateur thermique.

**12.** Récipient d'emballage médical selon la revendication 11, dans lequel le composant diol comprend en outre de l'éthylène glycol.

**13.** Récipient d'emballage médical selon l'une quelconque des revendications 1 à 12, comprenant une structure multicouche ayant au moins trois couches, et
une couche la plus interne et une couche la plus externe dans la structure multicouche comprenant la résine de polyester (A1).

**14.** Récipient d'emballage médical selon la revendication 13, dans lequel au moins une couche des couches intermédiaires dans la structure multicouche comprend une résine de polyoléfine (B).

**15.** Récipient d'emballage médical selon la revendication 14, dans lequel la résine de polyoléfine (B) est au moins une sélectionnée parmi un polymère de cyclooléfine, un copolymère de cyclooléfine, et du polypropylène.

**16.** Récipient d'emballage médical selon l'une quelconque des revendications 1 à 15, dans lequel le récipient d'emballage médical est une ampoule, un flacon, ou une seringue pré-remplie.

**17.** Procédé de fabrication d'une résine de polyester, comprenant les étapes consistant à :

produire un oligomère par transestérification d'un composant diester d'acide dicarboxylique comprenant un diester d'acide naphtalènedicarboxylique et au moins un sélectionné à partir des composés représentés par la

formule (1), la formule (2), et la formule (3), et un composant diol comprenant de l'éthylène glycol en présence d'un composé de manganèse ;et
polycondenser l'oligomère en présence d'un composé d'antimoine ;

dans lequel un composé de phosphore est utilisé comme un stabilisateur thermique, et
dans lequel la résine de polyester a une teneur en atomes de manganèse de 40 à 200 ppm et une teneur en atomes d'antimoine de 50 à 200 ppm, avec un rapport (M/P) de la teneur totale (M) (ppm) d'atomes de manganèse et d'atomes d'antimoine sur la teneur (P) (ppm) en atomes de phosphore de 1,5 à 4.

[Formule 6]

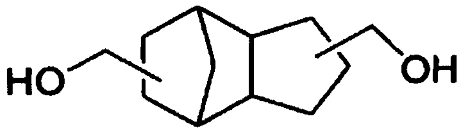

(1)

[Formule 7]

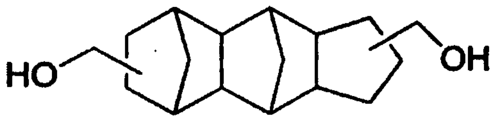

(2)

[Formule 8]

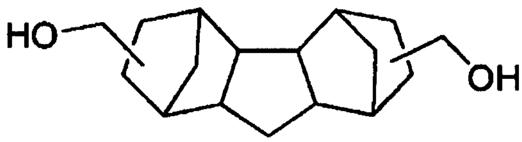

(3)

**18.** Procédé de fabrication de la résine de polyester selon la revendication 17, dans lequel les atomes de manganèse sont dérivés d'acétate de manganèse, les atomes d'antimoine sont dérivés de trioxyde d'antimoine, et les atomes de phosphore sont dérivés d'acide phosphorique.

**19.** Procédé de fabrication de la résine de polyester selon la revendication 17 ou 18, dans lequel le rapport d'éthylène glycol sur le total des composés représentés par la formule (1), la formule (2), et la formule (3) dans le composant diol est de 70 à 99 % en moles/30 à 1 % en moles.

**20.** Procédé de fabrication de la résine de polyester selon l'une quelconque des revendications 17 à 19, dans lequel le diester d'acide dicarboxylique est du diméthyle de 2,6-naphthalènedicarboxylate.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200875639 A
- JP 2004 A
- JP 298220 A
- JP 2001 A
- JP 240661 A
- JP 2003138074 A
- JP 8127641 A
- JP 2004229750 A
- JP 2007238856 A
- JP 58174419 A
- JP 2000504770 A
- JP 3014882 A
- JP 3122137 A
- JP 4063807 A
- JP 5300939 A
- JP 5317411 A
- JP 2004323058 A